(19) Europäisches Patentamt
European Patent Office
Office européen des brevets

(11) EP 2 186 819 A1

(12) EUROPEAN PATENT APPLICATION

(43) Date of publication:
**19.05.2010 Bulletin 2010/20**

(21) Application number: **10151082.4**

(22) Date of filing: **07.07.2006**

(51) Int Cl.:
***C07H 13/04*** *(2006.01)* ***C07D 309/06*** *(2006.01)*
***A61K 31/351*** *(2006.01)* ***A61K 31/341*** *(2006.01)*
***A61K 31/7028*** *(2006.01)* ***A61K 31/7024*** *(2006.01)*

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(30) Priority: **07.07.2005 DK 200501002**

(62) Document number(s) of the earlier application(s) in accordance with Art. 76 EPC:
**06753340.6 / 1 915 387**

(71) Applicant: **Aditech Pharma AG**
**6300 Zug (CH)**

(72) Inventors:
- **Nilsson, Henrik**
**8835, Feusisberg (CH)**
- **Andersen, Jens E. T.**
**2950, Vedbæk (DK)**

(74) Representative: **Stahr, Pia**
**Inspicos A/S**
**Kogle Allé 2**
**P.O. Box 45**
**2970 Hørsholm (DK)**

Remarks:
This application was filed on 19-01-2010 as a divisional application to the application mentioned under INID code 62.

(54) **Novel glucopyranose esters and glucofuranose esters of alkyl- fumarates and their pharmaceutical use**

(57) The present invention relates to novel glucofuranose esters and glucopyranose esters of alkyl-fumarates. The esters are suitable for use as active substances in the treatment of e.g. psoriasis or other hyperproliferative, inflammatory or autoimmune disorders.

EP 2 186 819 A1

## Description

### Field of the invention

The present invention relates to novel glucofuranose esters and glucopyranose esters of alkyl-fumarates. The esters are suitable for use as active substances in the treatment of e.g. psoriasis or other hyperproliferative, inflammatory or autoimmune disorders either alone or in combination with other pharmaceuticals such as e.g. another fumaric acid ester.

### Background of the invention

Fumaric acid esters, i.e. dimethylfumarate in combination with ethylhydrogenfumarate have been used in the treatment of psoriasis for many years. The combination is marketed under the tradename Fumaderm@. It is in the form of tablets intended for oral use and it is available in two different dosage strengths (Fumaderm® initial and Fumaderm®):

| | Fumaderm@ Initial | Fumaderm® |
|---|---|---|
| Dimethylfumarate | 30 mg | 120 mg |
| Ethylhydrogenfumarate, calcium salt | 67 mg | 87 mg |
| Ethylhydrogenfumarate, Magnesium salt | 5 mg | 5 mg |
| Etylhydrogenfumarate, Zinc salt | 3 mg | 3 mg |

The two strengths are intended to be applied in an individually based dose regimen starting with Fumaderm@ initial in an escalating dose, and then after e.g. three weeks of treatment switching to Fumaderm@. Both Fumaderm@ initial and Fumaderm@ are enteric coated tablets.

Another marketed composition is Fumaraat 120® containing 120 mg of dimethylfumarate and 95 mg of calcium monoethylfumarate (TioFarma, Oud-Beijerland, Netherlands). In a recent publication (Litjens et al. Br. J. Clin. Pharmacol. 2004, vol. 58:4, pp. 429-432), the pharmacokinetic profile of Fumaraat 120® is described in healthy subjects. The results show that a single oral dose of Fumaraat 120® is followed by a rise in serum monomethylfumarate concentration and only negligible concentrations of dimethylfumarate and fumaric acid is observed. The results indicate that dimethylfumarate is rapidly hydrolyzed to monomethylfumarate in an alkaline environment, but according to the authors not in an acid environment. As the composition is enteric coated, it is contemplated that the uptake of fumarate takes place mainly in the small intestine, where dimethylfumarate before uptake is hydrolysed to the monoester due to an alkaline environment or it may rapidly be converted due to esterases in the circulation. Furthermore, the study shows that $t_{max}$ and $c_{max}$ are subject to food effect, i.e. $t_{max}$ is prolonged (mean for fasted conditions is 182 min, whereas for fed conditions mean is 361 min) [lag time is 90 min for fasted and 300 min for fed] and $c_{max}$ is decreased (fasted: 0.84 mg/l, fed: 0.48 mg/l) by concomitant food-intake. Another study (Reddingius W.G. Bioanalysis and Pharmacokinetics of Fumarates in Humans. Dissertation ETH Zurich No. 12199 )1997) in healthy subjects with two tablets of Fumaderm@ P forte revealed $c_{max}$ values (determined as monoethyl- or monomethylfumarate) in a range from 1.0 to 2.4 $\mu$g/ml and a $t_{max}$ in a range of from 4.8 to 6.0 hours.

US 6,277,882 and US 6,355,676 disclose respectively the use of alkyl hydrogen fumarates and the use of certain fumaric acid mono alkyl ester salts for preparing micro tablets for treating psoriasis, psoriatic arthritis, neurodermatitis and enteritis regionalis Crohn. US 6,509,376 discloses the use of certain dialkyl fumarates for the preparation of pharmaceutical preparations for use in transplantation medicine or the therapy of autoimmune diseases in the form of micro tablets or pellets. US 5,424,332 discloses calcium, magnesium, zinc and iron salts of fumaric acid monoalkyl esters. US 6,359,003 discloses treatment of transplant rejection by selectively suppressing host-versus-graft reaction using monoalkyl fumarate or its salt. US 4,959,389 disclose compositions containing different salts of fumaric acid monoalkyl ester alone or in combination with dialkyl fumarate. The Case report "Treatment of disseminated granuloma annulare with fumaric acid esters" from BMC Dermatology, vol. 2, no.5, 2002, relates to treatment with fumaric acid esters. US 2004/0038889 discloses use of fumaric acid amides for the therapy of an autoimmune disease, mithchondrial diseases, and NF-kappaB mediated diseases and in transplantation medicine. WO 89/01830 discloses fumaric acid diamides and monoamides for treatment of psoriasis.

However, therapy with fumarates like e.g. Fumaderm® frequently gives rise to flushing and/or gastro-intestinal side effects such as e.g. fullness, diarrhea, upper abdominal cramps, flatulence and nausea.

Furthermore, the present commercially available product contains a combination of two different esters of which one of the esters (namely the ethylhydrogenfumarate which is the monoethylester of fumaric acid) is present in three different salt forms (i.e. the calcium, magnesium and zinc salt). Although each individual form may have its own therapeutic profile it would be advantageous to have a much simpler product, if possible, in order to obtain a suitable therapeutic effect.

Accordingly, there is a need to develop novel drug compounds of therapeutically or prophylactically active fumaric acid esters that provide an alternative and potentially improved treatment e.g. with a reduction in flushing and/or a reduction in gastro-intestinal related side effects upon oral administration and/or increased bioavailability.

## Summary of the invention

The present invention provides in one aspect new novel monosaccharide esters (in the following also named glucofuranose (I) or glucopyranose (II)) esters) of alkyl-fumarates of the general formula (I) or (II)

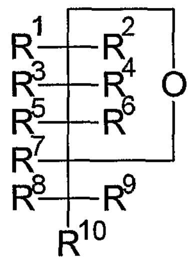

(I)

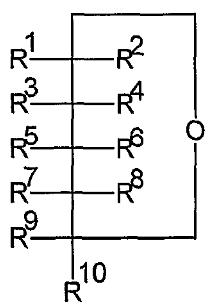

(II)

wherein $R^1$, $R^2$, $R^3$, $R^4$, $R^5$, $R^6$, $R^7$, $R^8$ and $R^9$ are the same or different and each is selected from the group consisting of hydrogen, hydroxyl, methoxy, methoxymethoxy, benzyloxy, acetate, acetonide and X, and

$R^{10}$ is selected from the group consisting of hydrogen, hydroxyl, $-CH_2OH$, $-CH_3$, X and $-CH_2$-X,

wherein

X is -OOC-CH=CH-COOR (trans) wherein R is $C_{1-5}$alkyl;

with the proviso that at least one of $R^1$, $R^2$, $R^3$, $R^4$, $R^5$, $R^6$, $R^7$, $R^8$, $R^9$ and $R^{10}$ is X or $R^{10}$ is $-CH_2X$,

and any enantiomers, racemates, diastereomers, anomers or tautomeric forms or mixtures thereof.

These novel drug compounds are contemplated to lead to an improved treatment of conditions susceptible to fumarate and/or fumaric acid ester treatment.

The mono- and dimethylester as well as the mono- and diethylester of fumaric acid have a poor solubility in water and this may be a factor leading to poor bioavailability (the bioavailability for the dimethylester of fumaric acid is regarded as very variable after oral administration). It is contemplated, that the monosaccharide esters according to the invention has the advantage that the monosaccharide part facilitates the absorption of the pharmaceutically active ingredient part in the intestine by the mechanisms that facilitate monosaccharide absorption, such as the so-called sodium co-transport and facilitated diffusion, possibly leading to an increased bioavailability.

Formation of the monosaccharideesters of alkyl-fumarates according to the invention may lead to a more suitable solubility in water or to a more suitable hydrophilic-lipophilic balance and, furthermore, due to the beneficial effect of the monosaccharide itself, the novel monosaccharide esters of alkyl-fumarates according to the invention are

contemplated to lead to an improved treatment regimen.

In further aspects, the invention relates to a pharmaceutical composition comprising a compound according to the invention.

In further aspects, the invention provides methods of treatment and use of said new monosaccharideesters of alkyl-fumarates in medicine and/or for combating tissue degenerative processes and/or more specifically in the treatment of conditions such as psoriasis, psoriatic arthritis, neurodermatitis, atopic dermatitis, inflammatory bowel disease, such as Crohn's disease and ulcerative colitis, autoimmune diseases such as polyarthritis, multiple sclerosis (MS), juvenile-onset diabetes, Hashimoto's thyroiditis, Grave's disease, SLE (systemic lupus erythematosus), Sjögren's syndrome, pernicious anemia, chronic active (lupoid) hepatitis, rheumatoid arthritis (RA) and optic neuritis, pain such as radicular pain, pain associated with radiculopathy, neuropathic pain or sciatica/sciatic pain; or for treatment of any of the following conditions: prevention of rejection following organ transplantation; sarcoidosis; necrobiosis lipoidica; and/or granuloma annulare, or for treatment of any of the following conditions: lupus nephritis, myasthenia gravis, uveitis, refractory uveitis, vernal conjunctivitis, pemphigus vulgaris, and/or scleroderma.

In another aspect of the invention, the use of said new monosaccharideesters of alkyl-fumarates for the manufacture of a pharmaceutical composition is provided. In another further aspect, pharmaceutical compositions are provided. In yet further aspects, methods for preparation of such new esters are provided.

**Disclosure of the invention**

In one aspect of the invention glucofuranose (I) or glucopyranose (II) esters of alkyl-fumarates of the general formula (I) or (II)

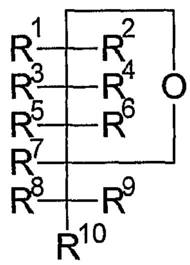

(I)

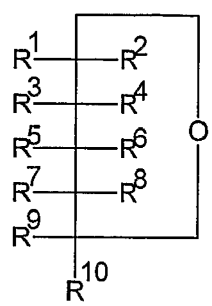

(II)

wherein $R^1$, $R^2$, $R^3$, $R^4$, $R^5$, $R^6$, $R^7$, $R^8$ and $R^9$ are the same or different and each is selected from the group consisting of hydrogen, hydroxyl, methoxy, methoxymethoxy, benzyloxy, acetate, acetonide and X, and $R^{10}$ is selected from the group consisting of hydrogen, hydroxyl, $-CH_2OH$, $-CH_3$, X and $-CH_2$-X,

wherein

X is -OOC-CH=CH-COOR (trans) wherein R is $C_{1-5}$alkyl;

with the proviso that at least one of $R^1$, $R^2$, $R^3$, $R^4$, $R^5$, $R^6$, $R^7$, $R^8$, $R^9$ and $R^{10}$ is X or $R^{10}$ is $-CH_2X$,

and any enantiomers, racemates, diastereomers, anomers or tautomeric forms or mixtures thereof, is provided.

In a further aspect of the invention, a compound of the general formula (I) or (II)

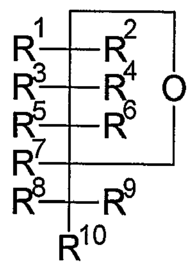

(I)

or

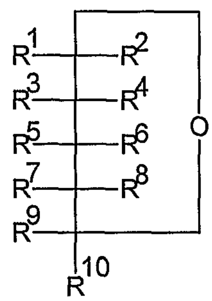

(II)

wherein $R^1$, $R^2$, $R^3$, $R^4$, $R^5$, $R^6$, $R^7$, $R^8$ and $R^9$ are the same or different and each is selected from the group consisting of hydrogen, hydroxyl and X, and

$R^{10}$ is selected from the group consisting of hydrogen, hydroxyl, $-CH_2OH$, $-CH_3$, X and $-CH_2$-X,

wherein

X is -OOC-CH=CH-COOR (trans) wherein R is $C_{1-5}$alkyl;

with the proviso that at least one of $R^1$, $R^2$, $R^3$, $R^4$, $R^5$, $R^6$, $R^7$, $R^8$, $R^9$ and $R^{10}$ is X or $R^{10}$ is $-CH_2X$, is provided.

In one aspect of the invention, the compound is of general formula I.

In another aspect of the invention, the compound is of general formula II.

The term "($C_{1-5}$)alkyl" or "$C_{1-5}$alkyl" refers to a straight-chained or branched alkyl group having from one to five carbon atoms inclusive such as methyl, ethyl, 1-propyl, 2-propyl, isopropyl, 1-butyl, 2-butyl, 2-methyl-2-propyl, 2-methyl-1-propyl, or pentyl. In a further aspect of the invention $R^1$ is methyl or ethyl, preferably methyl.

The term "-OOC-CH=CH-COOR (trans)" refers to

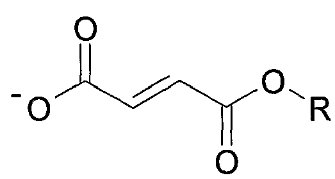

The term "hydroxyl" represents in the present context the radical -OH.

The term "methoxy" represents in the present context the radical $-OCH_3$.

The term "methoxymethoxy" represents in the present context the radical $-OCH_2OCH_3$.

The term "benzyloxy" represents in the present context the radical $-OCH_2Ph$.

The term "acetate" represents in the present context the radical $-OOCH_2CH_3$.

The term "acetonide" represents in the present context the radical $-OC(CH_3)_2O-$.

The compounds of the present invention may be chiral, and it is intended that any enantiomers, as separated, pure or partially purified enantiomers or racemic mixtures thereof are included within the scope of the invention.

In one aspect of the invention, the compound according to the invention is a D-enatiomer.

Furthermore, when a double bond or a fully or partially saturated ring system or more than one center of asymmetry or a bond is present with restricted rotability in the molecule, diastereomers may be formed. It is intended that any diastereomers, as separated, pure or partially purified diastereomers or mixtures thereof are included within the scope of the invention.

Furthermore, some of the compounds of the present invention may exist in different tautomeric forms and it is intended that any tautomeric forms, which the compounds are able to form, are included within the scope of the present invention.

Furthermore, some of the compounds of the present invention may exist in anomer forms and it is intended that any anomer form, which the compounds are able to form, are included within the scope of the present invention.

In one aspect of the invention, the compound according to the invention is a mixture of the $\alpha$- and the $\beta$- form. In a further aspect of the invention, the compound according to the invention is in the $\alpha$- form.

Accordingly, the present invention relates to novel monosaccharide esters of $C_{1-5}$alkyl-fumarates that may be used alone or in combination treatment e.g. with a di-$(C_{1-5})$alkylester of fumaric acid or other active substances.

The present invention also provides compositions including controlled release compositions comprising a novel ester according to the invention as well as the use of the novel esters in medicine. Furthermore, the present invention provides a method for the manufacturing of the novel esters according to the invention.

In one aspect of the invention, a composition according to the invention comprising a novel ester may - upon oral administration and in comparison to that obtained after oral administration of Fumaderm@ tablets in an equivalent dosage - give a reduction in GI (gastro-intestinal) related side-effects and/or reduce flushing (frequency and/or severity).

A suitable way of reducing the gastro-intestinal related side effects and/or flushing is likely to be by administration of a novel ester in the form of a controlled release composition.

As used in the present invention, a gastrointestinal (GI) side effect may include, but is not limited to diarrhea, stomach ache, stomach pain, abdominal pain, abdominal cramps, nausea, flatulence, tenesmus, meteorism, an increased frequency of stools, a feeling of fullness and upper abdominal cramps.

In the present context, a reduction of GI related side effects is intended to denote a decrease in severity and/or incidence among a given treated patient population, compared to the GI side effects observed after administration of the composition according to the invention compared with that of Fumaderm@. A reduction in GI related side effects according to this definition could thus be construed as a substantial reduction in incidence of any of the GI side effect listed above, such as at least a 10% reduction in incidence or more preferably at least 20 % reduction in incidence or even more preferably a more than 30 % reduction in incidence. A reduction in GI related side effect can also be expressed as a substantial reduction in severity in any of the GI side effects listed above, such as a reduction in severity and/or frequency of diarrhea, stomach ache, stomach pain, abdominal pain, abdominal cramps, nausea, flatulence, tenesmus, meteorism, increased frequency of stools, a feeling of fullness or upper abdominal cramps. The reduction of GI related side effects, as described above, can be monitored in a clinical trial setting, either comparing the administration of the composition according to the invention head on with Fumaderm@ or with placebo. In case of a placebo controlled trial, the incidence of GI related side effects in the patients receiving the composition according to the invention compared to the placebo group, can be compared to historical trials comparing Fumaderm@ to placebo (see e.g. Altmeyer et al, J. Am. Acad. Dermatol. 1994; full reference: Altmeyer PJ et al, Antipsoriatic effect of fumaric acid derivatives. Results of a multicenter double-blind study in 100 patients. J. Am. Acad. Dermatol. 1994; 30:977-81). Typically, patients suffering from psoriasis are included in such a study, and typically more than 10% of the body surface area will be affected by psoriasis (severe psoriasis). However, patients in whom between 2 and 10 percent of the body surface area is affected can also be included (moderate psoriasis). Patients can also be selected based on the psoriasis area severity index (PASI). Typically, patients within a certain range of PASI are included, such as between 10 and 40, or such as between 12 and 30, or such as between 15 and 25 or $\geq$10 or $\geq$12 or $\geq$16. Patients with any type of psoriasis may be included (chronic plaque type, exanthematic guttate type, pustular type, psoriatic erythroderma or palmoplantar type), but in some cases only patients with the chronic plaque type are included. About 15 to 20 patients in each treatment group (composition according to the invention and Fumaderm@ or placebo) are sufficient in most cases, but more preferably about 30 to 50 patients are included in each arm of the study. Total study duration can be as short as one day to one week, but more preferably the study will run for 8 weeks to 12 weeks or up to 16 weeks. The side effects can e.g. be assessed as the total number of times a certain side effect was reported in each group (irrespective of how many patients have experienced the side effect), or the side effects can be assessed as the number of patients that have experienced a certain side effect a certain number of times, such as at least once or at least twice or at least three times during the duration of the study. Furthermore, the severity of a side effect can be monitored, or a certain severity of a side effect can be required for it to qualify as a side effect in the study. A convenient way of assessing the severity of a side effect

is via a visual analogue (VAS) scale.

In the present context the term "flushing" describes episodic attacks of redness of the skin together with a sensation of warmth or burning of the face, neck, and less frequently the upper trunk and abdomen. It is the transient nature of the attacks that distinguishes flushing from the persistent erythema of photosensitivity or acute contact reactions. Repeated flushing over a prolonged period of time can lead to telangiectasia and occasionally to classical rosacea of the face (Greaves MW. Flushing and flushing syndromes, rosacea and perioral dermatitis. In: Champion RH, et al, eds. Rook/Wilkinson/Ebling textbook of dermatology, 6th ed., vol. 3. Oxford, UK: Blackwell Scientific, 1998: 2099-2104).

In the present context, a reduction of flushing is intended to denote a decrease in severity and/or incidence/frequency among a given treated patient population of flushing observed after administration of the composition according to the invention compared with flushing observed after administration of Fumaderm® and can be measured e.g as described by O'toole et al. Cancer 2000, 88(4): p. 770-776. A reduction in flushing according to this definition could thus be construed as a substantial reduction in incidence or severity of flushing. In one aspect of the invention, the incidence of flushing is reduced by at least about a third, in another aspect of the invention the incidence is reduced by half, and in a further aspect of the invention, the flushing incidence is reduced by about two thirds or more. Likewise, the severity is in one aspect of the invention reduced by at least about a third, in another aspect of the invention by at least half, and in a further aspect of the invention by at least about two thirds. Clearly a one hundred percent reduction in flushing incidence and severity is most preferable, but is not required. The reduction of flushing, as described above, can be monitored in a clinical trial setting, e.g. comparing the administration of the compound according to the invention compared with treatment with e.g. administration of Fumaderm®. In case of a Fumaderm® controlled trial, the incidence and severity, defined as mild, moderate or severe, of flushing in the patients receiving the compound according to the invention compared to the Fumaderm® group, can be compared. Typically, patients suffering from psoriasis are included in such a study, and typically more than 10% of the body surface area will be affected by psoriasis (severe psoriasis). However, patients in whom between 2 and 10 percent of the body surface area is affected can also be included (moderate psoriasis). Patients can also be selected based on the psoriasis area severity index (PASI). Typically, patients within a certain range of PASI are included, such as between 10 and 40, or such as between 12 and 30, or such as between 15 and 25 or ≥10 or ≥12 or ≥16. Patients with any type of psoriasis may be included (chronic plaque type, exanthematic guttate type, pustular type, psoriatic erythroderma or palmoplantar type), but in some cases only patients with the chronic plaque type are included. About 15 to 20 patients in each treatment group are sufficient in most cases, but more preferably about 30 to 50 patients are included in each arm of the study. Total study duration can be as short as one day to one week, but more preferably the study will run for 8 weeks to 12 weeks or up to 16 weeks. The side effects can e.g. be assessed as the total number of times a certain side effect was reported in each group (irrespective of how many patients have experienced the side effect), or the side effects can be assessed as the number of patients that have experienced a certain side effect a certain number of times, such as at least once or at least twice or at least three times during the duration of the study. Furthermore, the severity of a side effect can be monitored, or a certain severity of a side effect can be required for it to qualify as a side effect in the study. A convenient way of assessing the severity of a side effect is via a visual analogue (VAS) scale. Intestinal permeability of the compounds according to the invention may be determined using several different methods in the art. Intestinal permeability may be determined e.g. as described by Werdenberg et al. (BioPharm. Drug Dispos. 24: 259-273 (2003)) by isolated intestinal mucosa as well as by Caco 2 cell mono layers in order to obtain estimates of the fraction of the dose absorbed for these compounds (as also described in example 2).

*Glucopyranoses and glucofuranoses*

Monosaccharides may be found as ring-shaped structures where 4-5 carbon atoms and one oxygen atom form a five-membered ring or a six-membered ring. The five-membered ring and the six-membered ring are denoted as furanoses and pyranoses, respectively.

In the present context the monosaccharide is described as a ring system as shown in below Fischer-projections of general formula I and formula II

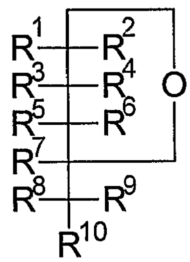

(I)

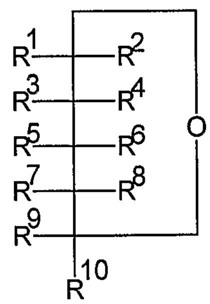

(II)

In common glucopyranoses and glucofuranoses, the radicals $R^1$-$R^9$ are distributed as 4 OH and 5 H and $R^{10}$ is hydrogen or $CH_2OH$. An ester of a monosaccharide is obtained, when one or more of the substituents $R^1$-$R^{10}$ are substituted by at least one ester group (i.e. one hydroxyl group is acylated) and the remaining substituents are either radicals H or OH or in a further aspect methoxy, methoxymethoxy, benzyloxy, acetate or acetonide. In one aspect according to the invention, five or six of $R^1$, $R^2$, $R^3$, $R^4$, $R^5$, $R^6$, $R^7$, $R^8$, $R^9$ and $R^{10}$ are hydrogen. The monosaccharides could in one aspect of the invention be the alfa-D forms or the beta-D forms or mixtures thereof of furanose comprising allofuranose, altrofuranose, arabinofuranose, erythrofuranose, galactofuranose, glucofuranose, gulofuranose, idofuranose, lyxofuranose, mannofuranose, psicofuranose, ribofuranose, ribulofuranose, sorbofuranose, tagatofuranose, talofuranose, threofuranose, xylofuranose, xylulofuranose or fructofuranose.

Alternatively, the monosaccharides could be either the alfa-D forms or the beta-D forms or mixtures thereof of pyranose comprising allopyranose, altropyranose, arabinopyranose, erythropyranose, galactopyranose, glucopyranose, gulopyranose, idopyranose, lyxopyranose, mannopyranose, psicopyranose, ribopyranose, ribulopyranose, sorbopyranose, tagatopyranose, talopyranose, threopyranose, xylopyranose, xylulopyranose or fructopyranose.

In a further aspect of the invention the monosaccharide part of the compound according to the invention is arabinofuranose, galactofuranose, glucofuranose, mannofuranose, xylofuranose, fructofuranose arabinopyranose, galactopyranose, glucopyranose, mannopyranose, xylopyranose or fructopyranose.

In one aspect of the invention, in a monosaccharide up to five hydroxyls could be substituted by an alkoxide group and all remaining radicals are H.

The reaction between e.g. monomethyl fumarate (MMF) and a monosaccharide would yield an ester of this general type, with an ester bond to one or more of the OH positions of the monosaccharide. In order to ensure synthesis of a single homogenous product, the stereochemistry of the carbons must be retained after reaction with the OH-groups attached to the carbons. A single well defined product may be obtained by protection of individual OH-groups with methoxys, methoxymethoxys, benzyloxy's, acetates or by protection of two OH-groups using acetonides, which ensures reaction with MMF at the desired number of carbons in the monosaccharide e.g. at a single-carbon position of the monosaccharide.

*Active substance*

In an aspect of the invention, the fumaric acid ester is a mono-($C_{1-5}$)alkylester of fumaric acid that is present in the form of a monosaccharideester of alkyl-fumarate according to general formula (I) or (II).

The active substance in a composition of the invention is a monosaccharide ester of a fumaric acid monoalkylester such as monomethylfumarate, monoethylfumarate, monopropylfumarate, monobutylfumarate and monopentylfumarate.

In one aspect of the invention, the compound is selected from the group consisting of arabinofuranosyl-$C_{1-5}$alkyl-fumarate, galactofuranosyl-$C_{1-5}$alkyl-fumarate, glucofuranosyl-$C_{1-5}$alkyl-fumarate, mannofuranosyl-$C_{1-5}$alkyl-fumarate, xylofuranosyl-$C_{1-5}$alkyl-fumarate, fructofuranosyl-$C_{1-5}$alkyl-fumarate, arabinopyranosyl-$C_{1-5}$alkyl-fumarate, galacto-pyranosyl-$C_{1-5}$alkyl-fumarate, glucopyranosyl-$C_{1-5}$alkyl-fumarate, mannopyranosyl-$C_{1-5}$alkyl-fumarate, xylopyranosyl-$C_{1-5}$alkyl-fumarate and fructopyranosyl-$C_{1-5}$alkyl-fumarate. In still another aspect the $C_{1-5}$alkyl is methyl.

In one aspect of the invention, the compound according to the invention is of general formula I.

In a further aspect of the invention, the compound according to the invention is of general formula II.

In a further aspect of the invention, the compound according to the invention is a D-alpha anomer or D-beta anomer, or mixtures thereof.

In a further aspect of the invention, the compound according to the invention is a D-alpha anomer.

In a further aspect of the invention, five or six of $R^1$, $R^2$, $R^3$, $R^4$, $R^5$, $R^6$, $R^7$, $R^8$, $R^9$ and $R^{10}$ are hydrogen.

In a further aspect of the invention, R is methyl.

In a further aspect of the invention, $R^2$ is hydroxyl.

In a further aspect of the invention, $R^{10}$ is $-CH_2$-X.

In a further aspect of the invention, $R^1$ is hydrogen.

In a further aspect of the invention, either $R^3$ or $R^4$ is hydrogen and the other is hydroxyl.

In a further aspect of the invention, either $R^5$ or $R^6$ is hydrogen and the other is hydroxyl.

In a further aspect of the invention, either $R^7$ or $R^8$ is hydrogen and the other is hydroxyl.

The order of preference for the transport of different monosaccharides through the intestinal membrane and their relative rates of transport in comparison with glucose is galactose, glucose, fructose, mannose, xylose and arabinose. The compounds according to the invention are contemplated to be transported in the same order of preference.

In a further aspect of the invention, the compound is selected from the group consisting of

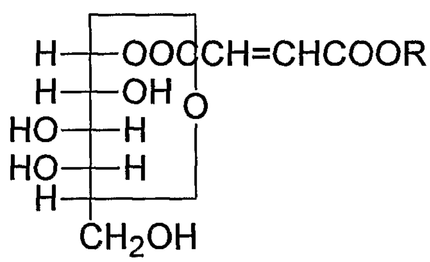

α-D-galactopyranosyl-alkylfumarate

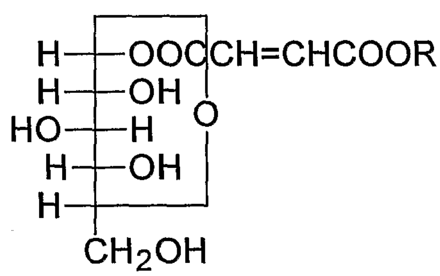

α-D-glucopyranosyl-alkylfumarate

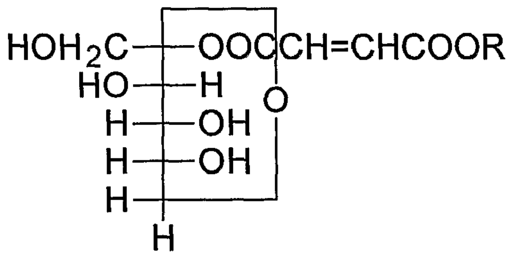

α-D-fructopyranosyl-alkylfumarate

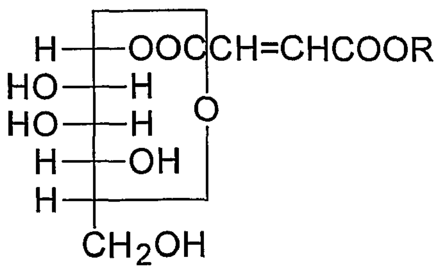

α-D-mannopyranosyl-alkylfumarate

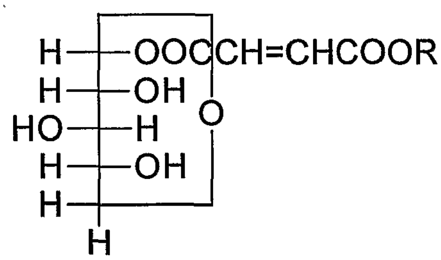

α-D-xylopyranosyl-alkylfumarate

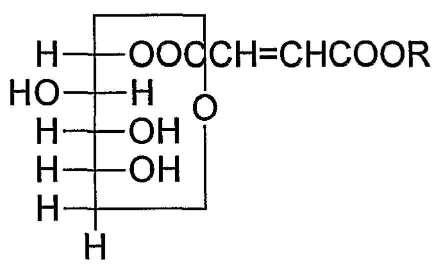

α-D-arabinopyranosyl-alkylfumarate

wherein R is $C_{1-5}$alkyl.

In a further aspect of the invention, the compound is selected from the group consisting of

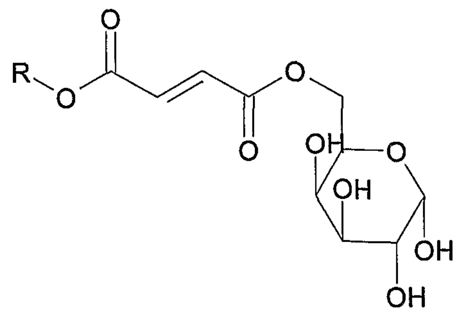

α-D-galactopyranose, 6-( $C_{1-5}$alkyl fumarate)

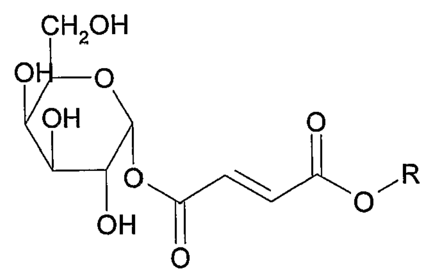

α-D-galactopyranose, 1-( $C_{1-5}$alkyl fumarate)

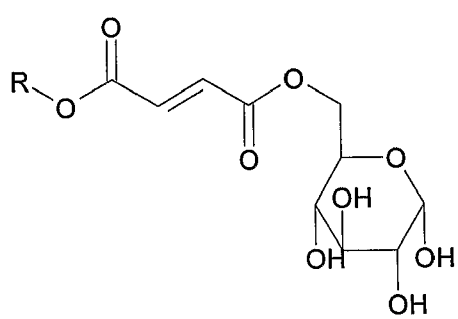

α-D-glucopyranose, 6-( $C_{1-5}$alkyl fumarate)

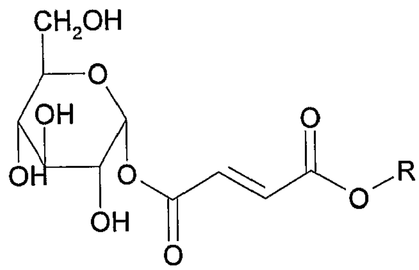

α-D-glucopyranose, 1-( $C_{1-5}$alkyl fumarate)

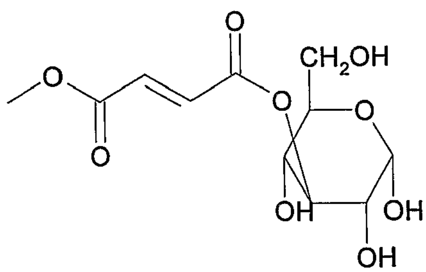

α-D-glucopyranose, 3-( $C_{1-5}$alkyl fumarate)

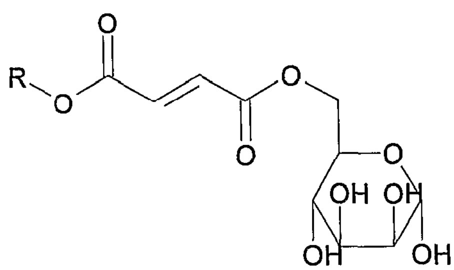

α-D-mannopyranose, 6-( $C_{1-5}$alkyl fumarate)

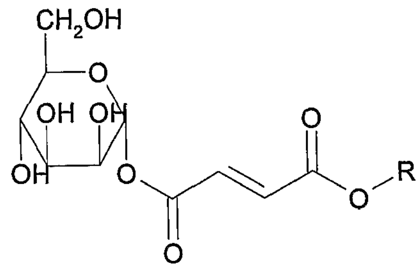

α-D-mannopyranose, 1-( $C_{1-5}$alkyl fumarate)

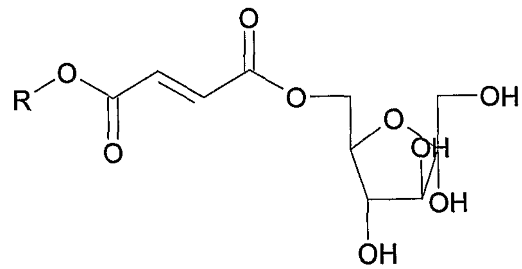

α-D-fructofuranose,6-( $C_{1-5}$alkyl fumarate)

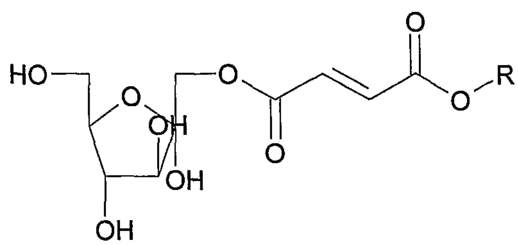

α-D-fructofuranose,1-( $C_{1-5}$alkyl fumarate)

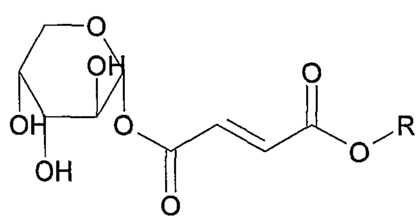

α-D-arabinopyranose,1-( $C_{1-5}$alkyl fumarate)

and

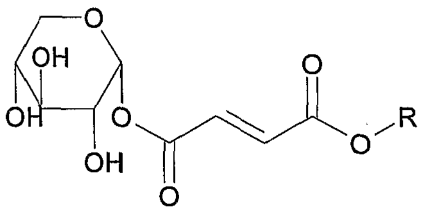

α-D-xylopyranose,1-( $C_{1-5}$alkyl fumarate)

wherein R is $C_{1-5}$alkyl.

In a further aspect, R in above compounds is methyl.

The active substance may be used in combination with another active fumaric acid ester such as a dialkylfumarate like e.g. dimethylfumarate, diethylfumarate, dipropylfumarate, dibutylfumarate, dipentylfumarate, methyl-ethylfumarate, methyl-propylfumarate, methyl-butylfumarate or methyl-pentylfumarate, or monoalkylfumarates such as monomethylfumarate, monoethylfumarate, monopropylfumarate, monobutylfumarate or monopentylfumarate including pharmaceutically acceptable salts thereof.

In another aspect, a composition according to the invention comprises a monosaccharideester of $C_{1-5}$alkylfumarate according to the invention together with a di($C_{1-5}$)alkylester of fumaric acid (e.g. dimethylfumarate) as the active substances.

In a further aspect, the composition according to the invention comprises as active substances a combination of a monosaccharideester of $C_{1-5}$alkyl-fumarate according to the invention and a mono($C_{1-5}$)alkylester of fumaric acid (e.g. monomethylfumarate) optionally in the form of a pharmaceutically acceptable salt like e.g. its sodium, potassium, strontium, calcium, magnesium and/or zinc salt.

*Synthesis of monosaccharideesters of $C_{1-5}$alkyl-fumarate according to the invention*

Fumaric acid, its monomethyl ester and its dimethylester are well known compounds that may be isolated from plants or synthesized (K.S. Rao and S.H. Mishra, J. Ethnopharmacology, vol. 60 (3), a998, pp. 207-213). The synthesis of the monomethyl ester of fumaric acid is not necessarily straightforward because of symmetry. Accordingly, attempts to synthesize the monomethyl ester by adding methanol to fumaric acid may inevitably lead to formation of the dimethyl ester. In addition, the synthesis may be complicated by the presence of the double bond, which under elevated temperature and pressure may hydrolyse and produce oxalic acid. The monomethyl fumarate may be synthesised by hydrolysis of methyl hydrogen fumarate following the method by Spatz and Stone (J. Org. Chem., vol. 23 (10), 1958, pp. 1559-1560). Several ways of producing the monosaccharideesters of $C_{1-5}$alkyl-fumarate according to the invention may be contemplated.

In an aspect of the invention, a method for preparing a monosaccharide-C $_{1-5}$alkyl-fumarate according to the invention is provided, comprising synthesis along two pathways.

(1) In the first approach, a pyranoside or a furanoside is formed by reaction of pyranose or furanose, respectively, with a protection group, such as methoxy, methoxymethoxy, benzyloxy, acetate or acetonide, that prevents degradation and selective derivatization of the carbohydrate. A β-D-glucosyl ester of indole-3-acetic acid is formed by a straightforward procedure where a fully benzylated 1-O-glucosylpseudourea intermediate is reacted with indole-3-acetic acid, followed by hydrogenolytic removal of the protective groups (A. Jakas, V. Magnus, S. Horvat and G. Sandberg, J. Labell. Comp. Pharm. 33(10) (1993) pp. 933-939).

(2) In a second approach, the unprotected pyranose or the unprotected furanose is reacted selectively to form the desired ester compounds by enzymatically catalyzed reactions with suitable carboxylic acids. Regioselective synthesis of L-phenylalanyl ester of D-glucose with unprotected L-phenylalanine and D-glucose is performed in organic solvents using lipase assays (K. Lohith, S. Divakar, J. Biotechnol. 117 (2005) 49-56).

*Dosage*

Apart from providing pharmaceutical compositions having different content of the compounds according to the invention present, the invention in one aspect also provides kits containing two or more containers e.g. with compositions having various amounts of the compounds according to the invention included. Such kits are e.g suitable for use in those situations where an increasing dosage is required over time.

In one aspect of the invention, an up-scale of the dosage is e.g. ½ dose for 3-7 days, such as 7 days, thereafter full dose, alternatively 1/3 of the dose for 3-7 days such as 7 days, thereafter 2/3 of the dose for 3-7 days such as 7 days, thereafter full dose, alternatively full dose from day one.

In one aspect of the invention, a pharmaceutical composition wherein the amount of monosaccharideester of $C_{1-5}$alkyl-fumarate in a dosage form is from 90 mg to 1000 mg active substance, such as 90 mg to 600 mg active substance, such as 90 mg to 540 mg active substance, such as 90 mg to 500 mg active substance, such as 90 mg to 360 mg active substance, such as 90, 120, 180, 240, 360, 450, 480, 500, 540, 600 or 1000 mg active substance, is provided. In a further aspect of the invention the amount of active substance is 120, 180 or 240 mg active substance. In yet a further aspect of the invention, the amount of active substance is 180 or 360 mg.

The daily dosage of the pharmaceutical composition according to the invention that is administered to treat a patient depends on a number of factors among which are included, without limitation, weight and age and the underlying causes of the condition or disease to be treated, and is within the skill of a physician to determine. In one aspect of the invention the daily dosage can be e.g. from 240 to 360 mg active substance given in one to three doses, in another aspect from 360 to 480 mg active substance given in one to three doses, in another aspect 480 to 600 mg active substance given in one to three doses, in another aspect 600 to 720 mg active substance given in one to three doses, in another aspect 720 to 840 mg active substance given in one to three doses, in another aspect 840 to 960 mg active substance given in one to three doses and in yet another aspect 960 to 1080 mg active substance given in one to three doses.

In another aspect of the invention, a pharmaceutical composition in the form of a tablet is provided, such as a tablet which has a shape that makes it easy and convenient for a patient to swallow e.g. a tablet which has a rounded or a rod-like shape without any sharp edges.

In another aspect of the invention, a pharmaceutical composition in the form of a tablet designed to be divided into two or more parts, is provided.

The compositions according to the invention may be administered together with a meal or in relation to a meal such as e.g. in a time period corresponding to a range from at least about 30 minutes before a meal to about 2 hours after the meal, or the composition may be administered at any specific point(s) in time during the day.

In one embodiment, the total daily dose is given at bedtime, such as up to or about 30 minutes before bedtime, up to or about 60 minutes before bedtime, up to or about 90 minutes before bedtime, up to or about 120 minutes before bedtime or up to or about 180 minutes before bedtime.

In one aspect of the invention, the dosage of a compound according to the invention to be administered should provide a peak plasma concentration ($C_{max}$) of the corresponding alkyl fumarate in a range of from about 0.4 to about 4.0 mg $l^{-1}$ after a single dose administration to humans, such as from about 0.5 to about 3.0 mg $l^{-1}$ after a single dose administration to humans, such as from about 1.0 to about 2.5 mg $l^{-1}$ after a single dose administration to humans, such as from about 1.0 to about 2.0 mg $l^{-1}$ after a single dose administration to humans.

In another aspect of the invention, the dosage of a compound according to the invention to be administered should provide an area under the plasma concentration vs. time profile ($AUC_{0-\infty}$) of the corresponding alkyl fumarate of from about 30 to 750, such as from about 30 to 600, from about 30 to 450, from about 30 to 300 or from about 30 to 150 mg x min $l^{-1}$ after a single dose administration to humans.

In another aspect of the invention, the total daily dosage of a compound according to the invention to be used should provide a clinical effect as measured by the percentage of subjects achieving a PASI 75 (a PASI reduction of ≥75% from baseline PASI) after 12 weeks of treatment of at least 20%, such as at least 30%, such as at least 40%, such as at least 50%, such as at least 60%, such as about 40%, such as about 50%.

In another aspect of the invention, the total daily dosage of a compound according to the invention to be used should provide a clinical effect as measured by the percentage of subjects achieving a PASI 75 (a PASI reduction of ≥75% from baseline PASI) after 16 weeks of treatment of at least 20%, such as at least 30%, such as at least 40%, such as at least 50%, such as at least 60%, such as about 40%, such as about 50%.

In another aspect of the invention, the total daily dosage of a compound according to the invention to be used should provide a clinical effect as measured by the percentage of subjects achieving a PASI 75 (a PASI reduction of ≥75% from baseline PASI) after 24 weeks of treatment of at least 30%, such as at least 40%, such as at least 50%, such as at least 60%, such as at least 70%, such as about 40%, such as about 50%, such as about 60%.

The clinical effect of the compounds according to the invention may be measured in a double-blind, placebo controlled, parallel-group study. Eligible patients for testing for the effect on e.g. psoriasis are e.g. patients who have

had psoriasis (chronic, exanthematic guttate, erythrodermic, plamoplantar, or pustular) for at least 1 year. Patients should typically have a baseline PASI of 16-24or ≥10, or ≥12. Systemic treatment should be discontinued 4 weeks before study initiation. Topical treatment should be discontinued 2 week before study initiation. Only topical salicylic acids and emollients should be allowed during the study period.

Patients should be randomised to either the placebo-group or to a group receiving the pharmaceutical composition according to the invention.. The total number of patients to be included will depend on the specific study-design but may be e.g. 80 patients with 40 patients on placebo and 40 patients on active treatment.

The treatment period is 12-16 weeks or up to 24 weeks, or up to 52 weeks. The primary measure of efficacy is the reduction in PASI score between baseline and at the end of treatment, or the percentage of subjects achieving e.g. a PASI 75 (≥75% reduction from baseline in their PASI scores) or a PASI 90 (≥90% reduction from baseline in their PASI scores), or by determining the change in the physician's global assessment (PGA).

In one aspect of the invention, the compounds according to the invention have an increased dissolution rate leading to increased bioavailability compared to administration of monomethyl fumarate. In another aspect of the invention, the compounds according to the invention have an increased bioavailability in-vivo as compared to the administration of monomethyl fumarate.

Uses

The term "treatment" as used herein means the management and care of a patient for the purpose of combating a disease, disorder or condition. The term is intended to include the delaying of the progression of the disease, disorder or condition, the alleviation or relief of symptoms and complications, and/or the cure or elimination of the disease, disorder or condition. The patient to be treated is preferably a mammal, in particular a human being.

The terms "disease", "condition" and "disorder" as used herein are used interchangeably to specify a state of a patient which is not the normal physiological state of man.

The compounds, compositions and kits according to the invention are contemplated to be suitable for use in medicine and/or for combating tissue degenerative processes, hyperproliferative, inflammatory and/or autoimmune disorders
and more specifically for the treatment of one or more of the following conditions:

a. Psoriasis

b. Psoriatic arthritis

c. Neurodermatitis, atopic dermatitis

d. Inflammatory bowel disease, such as

    i. Crohn's disease

    ii. Ulcerative colitis

e. Autoimmune diseases:

    i. Polyarthritis

    ii. Multiple sclerosis (MS)

    iii. Juvenile-onset diabetes mellitus

    iv. Hashimoto's thyroiditis

    v. Grave's disease

    vi. SLE (systemic lupus erythematosus)

    vii. Sjögren's syndrome

    viii. Pernicious anemia

ix. Chronic active (lupoid) hepatitis

x. Rheumatoid arthritis (RA)

xi. Optic neuritis

f. Pain such as radicular pain, pain associated with radiculopathy, neuropathic pain or sciatica/sciatic pain

g. Organ transplantation (prevention of rejection)

h. Sarcoidosis

i. Necrobiosis lipoidica

j. Granuloma annulare

Moreover, the novel compounds, compositions or kits according to the invention may be used in the treatment of one or more of the following conditions lupus nephritis, myasthenia gravis, uveitis, refractory uveitis, vernal conjunctivitis, pemphigus vulgaris, and/or scleroderma.

Psoriasis has been proposed to potentially be associated with Crohn's disease (Najarian DJ, Gottlieb AB, Connections between psoriasis and Crohn's disease. J Am Acad Dermatol. 2003 Jun;48(6):805-21), celiac disease (Ojetti V et al, High prevalence of celiac disease in psoriasis. Am J Gastroenterol. 2003 Nov;98(11):2574-5.), psychiatric or psychological disease, such as depression or a life crisis (Gupta MA, Gupta AK, Psychiatric and psychological co-morbidity in patients with dermatologic disorders: epidemiology and management. Am J Clin Dermatol. 2003;4(12): 833-42. and Mallbris L et al, Psoriasis phenotype at disease onset: clinical characterization of 400 adult cases. J Invest Dermatol. 2005 Mar;124(3):499-504.), overweight, diabetes mellitus, excess consumption of alcohol/alcoholism, as well as psoriatic arthritis.

The present invention thus relates in one aspect to a method of treating one or more conditions selected from the group consisting of psoriasis, psoriatic arthritis, neurodermatitis, inflammatory bowel disease, such as Crohn's disease and ulcerative colitis, autoimmune diseases such as polyarthritis, multiple sclerosis (MS), juvenile-onset diabetes mellitus, Hashimoto's thyroiditis, Grave's disease, SLE (systemic lupus erythematosus), Sjögren's syndrome, Pernicious anemia, Chronic active (lupoid) hepatitis, Rheumatoid arthritis (RA), and optic neuritis, pain such as radicular pain, pain associated with radiculopathy, neuropathic pain or sciatica/sciatic pain, organ transplantation (prevention of rejection), sarcoidosis, necrobiosis lipoidica, granuloma annulare, lupus nephritis, myasthenia gravis, uveitis, refractory uveitis, vernal conjunctivitis, pemphigus vulgaris, and scleroderma, which method comprises administering orally to a patient in need thereof, an effective dosage of a of a compound according the invention.

The present invention relates in another aspect to the use of a compound according to the invention for the preparation of a medicament for the treatment of one or more conditions selected from the group consisting of psoriasis, psoriatic arthritis, neurodermatitis, inflammatory bowel disease, such as Crohn's disease and ulcerative colitis, autoimmune diseases such as polyarthritis, multiple sclerosis (MS), juvenile-onset diabetes mellitus, Hashimoto's thyroiditis, Grave's disease, SLE (systemic lupus erythematosus), Sjögren's syndrome, Pernicious anemia, Chronic active (lupoid) hepatitis, Rheumatoid arthritis (RA), and optic neuritis, pain such as radicular pain, pain associated with radiculopathy, neuropathic pain or sciatica/sciatic pain, organ transplantation (prevention of rejection), sarcoidosis, necrobiosis lipoidica, granuloma annulare, lupus nephritis, myasthenia gravis, uveitis, refractory uveitis, vernal conjunctivitis, pemphigus vulgaris, and scleroderma.

In one aspect of the invention, a compound according to the invention for use in the treatment of one or more conditions, where the condition is selected from psoriasis, psoriatic arthritis, neurodermatitis and multiple sclerosis (MS), is provided. In yet a further aspect of the invention, a compound according to the invention for use in the treatment of psoriasis, is provided.

In yet a further aspect of the invention, a compound according to the invention for use in the treatment of multiple sclerosis, is provided.

In yet a further aspect of the invention, a compound according to the invention for use in the treatment of psoriatic arthritis, is provided.

Furthermore, the invention also relates to treating an individual suffering from one of the conditions in the abovementioned lists, more specifically multiple sclerosis, with a compound, composition or kit according to the invention, said individual further being in treatment with one or several compounds selected from the group consisting of PDB-0921 (P-005063, P-005088, P-005291, PDB-5613, PDB-5792), BGC-20-884, atorvastatin, Abatacept, alemtuzumab, Sativex, daclizumab, glatiramer acetate, ibudilast, interferon (Serono (\b1a)), interferon (AW (\a)), interferon (Biogen

(\b1a)), interferon (Novartis (\b1b)), interferon (Hemispherx), alefacept, levetiracetam, memantine hydrochloride, mitoxantrone, rituximab, simvastatin, baclofen (intrathecal), Cannabis (SIMM-18), Corticotropin, MLN-3897, MLN-519 (LDP-519, PN-05, PS-519), AEG-35156 (AEG-161, AEG-35169, GEM-640), RG-2077 (CTLA4-Ig, RG-1059), TBC-4746, MMP-12 inhibitors (Serono), R-1295, TRX-1, CDP-323, SC-12267, MDX-1100, ACE inhibitors (GenoMed), Cannabinor (PRS-211375), AVE-9897, JNK inhibitors (Serono), TV-3606, MLN-3701, rHDL (ZLB, CSL), AGT-1, NeuroVax (AI-208, BV-13S1, BV-5S2, BV-6S5, IR-208), fontolizumab, atiprimod dimaleate (Symadex), IP-751 (ajulemic acid, CT-3, DMH-11C), IDN-6556, Talampanel (GYKI-53773, LY-293606, LY-300164), GPI-1485 (GPI-1005, GPI-1046, GPI-1152, GPI-1216), talotrexin ammonium, AVR-118, Onercept, merimepodib, ABT-874, laquinimod, APT-070C, interferon (Pepgen (tau), Tauferon), IL-18BP (Yeda), ISIS-107248 (ATL-1102), delmitide acetate, SGN-30, MM-093 (ABI.001), AMG-487 (CCX-395, T-487), Monarsen (EN-101), EMZ-701, INO-1001, chaperonin-10 (CBio, Cpn-10), INCB-003284, STA-5326, Tovaxin, MLN-1202, BHT-3009, c-6448, Aimspro, RPI-78M, JM-002, Peptide T (Advanced Immuni T), TV-5010, N-palmitoylethanolamide (Stief), E3 (Effective Pharmaceuticals), 808-2, FAR-404, MCT-215, MK-0812, GEM-SP, Pixantrone (BBR-2778), Dexanabinol (HU-211, PRS-211092, PRS-211095, PRS-211220), fingolimod hydrochloride (FTY-720, FTY-720A, FTY-726), fampridine-SR, pirfenidone, Theralux, temsirolimus, E-2007, teriflunomide, MBP-8298, interferon (Rentschler (\b)-2), CNTO-1275, cladribine (IVAX), HumaT4 (anti-CD4 MAb, Intracel), MV-57471, glial growth factor-2 (CeNeS), M1 MAbs (Acorda), neural stem cells (StemCells), stem cells (hESCs, Geron), CXCR3 antagonists (Pharmacopeia), SCS technology, Pharmaprojects No. 5480, Immunosuppressants (p53-69, GPC), NBI-59159, E-2050 (ER-129002-02), neuregulin-2 (Acorda), soluble CD8 (MiDex , Avidex), IBD gene therapy (AMT), DN-1921 (Dantes), VLA-4 antagonists (Uriach), MS therapy (sodium channel blocker, Genopia), Erythropoietin (WP-170, Warren), heparanase inhibitors (Progen), CD-200Fc, MS therapy (MHC inhibitors, Provid), SGN-35, Neliximab, SYN-5001, interferon (Syntonix (\b)), PP-0102, LOR-S03, CCX-634, TMC-2003, MRK-167 (CMPD-167), TNF-\a inhibitors (Xencor), ReaDex, PLX-647, inflamm/autolmmune ther (Mann), SPR-1401, Antidepressants (ND-1251, ND-1510, Neuro3d), PXS-64 (PXS-25), PXS-2000, AT-008, autoimmune disease ther (Alnyl), interferon (Nautilus (\b)), CO-14, hedgehog agonists (neurological), anti-IL-23 (Archemix), BGC-20-0134, MORAb-022, MIF inhibitor (Genzyme), INCB-3344, immune regulating hormones (Hol), NNZ-2566, NNZ-4921, RX-111 (BL-1030), CLT-001, BKT-104, PEG-IFN-\b (Enzon), AZD-5904, interferon (Bolder (\b)), CB-2 agonists, BTG, Kv1.3 channel blockers (4SC), PS-375179, CCX-915, vitamin D signal amplifiers-5, Scleroneurin, IGIV (Hemosol), inflamm/autoimmune ther (Apollo), QR-442, leupeptin + taurine (C-201, Neurodur, CepTor), anti-CD3 antibody (Diversa), MLN-0415, Rob-895, AZD-8797, CHR-1103, multiple sclerosis ther (Brain), interferon (Vakzine (\b)), CCR2 antagonists (Merck & Co), GEMS-001, Natalizumab, BG-12 (Panaclar),and mitoxantrone.

In another embodiment, the invention relates to treating an individual suffering from one of the conditions in the abovementioned lists, more specifically multiple sclerosis, with a compound, composition or kit according to the invention, said individual further being in treatment with one or several compounds selected from the group consisting of beta-interferon 1a, beta-interferon 1b, natalizumab, BG-12, glatiramer acetate, mitoxantrone and fingolimod hydrochloride (FTY-720, FTY-720A, FTY-726).

Furthermore, the invention also relates to treating an individual suffering from one of the conditions in the abovementioned lists, more specifically psoriasis or psoriatic arthritis, with a compound, composition or kit according to the invention, said individual further being in treatment with

a) a topical anti-psoriatic drug such as 1) vitamin D or derivatives thereof (calcipotriol, calcipotriene), 2) a corticosteroid (such as e.g. betamethasone, desoximethasone, fluocinolone, momethasone, hydrocortisone aceponate, fluticasone, clobethasol, clobethasone, hydrocortisone butyrate, desonide, triamcinolone or hydrocortisone), 3) tazaroten, 4) ditranol, 5) tacrolimus (FK-506) and other calcineurin inhibitors, such as pimecrolimus or 6) any combination of 1-5 and/or

b) an oral anti-psoriatic drug such as 1) an oral retinoid (such as acitretin or etretinate) combined or not combined with PUVA, 2) cyclosporine and other calcineurin inhibitors, such as ISA247, tacrolimus and pimecrolimus, 3) methotrexate, 4) hydroxyurea, 5) azathioprine, 6) sulphasalazine, 7) a fumarate derivative (such as e.g. Fumaderm or BG-12), 8) rosiglitazone (Avandia) and other peroxisome proliferator-activated-$\gamma$ (PPAR$\gamma$) agonists or modulators, such as pioglitazone, farglitazar, GW1929, GW7845, MC-555, MBX-102/MBX-10, MBX-1828, MBX-2044, CLX-0921, R-483, reglitazar, naveglitazar (LY-519818/LY-818), netoglitazone (MCC-555), CS-7017, troglitazone, ciglitazone, tesaglitazar, isaglitazone, balaglitazone, muraglitazar, TAK-654, LBM642, DRF 4158, EML 4156, T-174, TY-51501, TY-12780, VDO-52 or AMG-131(T131) or any combination of 1-8 and/or

c) a parenterally administered anti-psoriatic drug such as 1) alefacept (Amevive), 2) etanercept (Enbrel), 3) efalizumab (Raptiva), 4) onercept, 5) adalimumab (Humira) or any combination of 1-5 and/or

d) an inhibitor of TNF-$\alpha$ not mentioned in the list under section c) above (e.g. CDP 870 or infliximab (Remicade)), administered via an enteral or parenteral route and/or

e) tisocalicitrate and/or NCX 1022 and/or IDEC-131 and/or MEDI-507, and/or

f) An NSAID or a COX or a LOX inhibitor such as e.g. a COX-2 inhibitor or a COX/5-LOX inhibitor, and/or

g) an anti-diabetic or anti-obesity drug, such as biguanides such as metformin; metformin XR; a sulphonylurea such as chlorpropamide, glipizide, gliciazide, glyburide/glibenclamide or glimepiride; Glucovance (metformin + glyburide); Metaglip (glipizide + metformin); a peroxisome proliferator-activated-γ (PPARγ) agonist or modulator, such as rosiglitazone (Avandia), pioglitazone, farglitazar, GW1929, GW7845, MC-555, MBX-102/MBX-10, MBX-1828, MBX-2044, CLX-0921, R-483, reglitazar, naveglitazar (LY-519818/LY-818), netoglitazone (MCC-555), CS-7017, troglitazone, ciglitazone, tesaglitazar, isaglitazone, balaglitazone, muraglitazar, TAK-654, LBM642, DRF 4158, EML 4156, T-174, TY-51501, TY-12780, VDO-52 or AMG-131(T131); Avandamet (rosiglitazone + metformin); Actos (pioglitazone + metformin); Avandaryl (rosiglitazone maleate + glimepiride); a benzoimidazole such as FK-614; CS-917; TA-1095; ONO-5129; TAK-559; TAK-677/AJ-9667; a d-phenylalanine inducer such as senaglinide; c-3347; NBI-6024; ingliforib; BVT 3498; LY 929; SGLT2 inhibitors; CS 011; BIM 51077; R1438; R1439; R1440; R1498; R1499; AVE 0847; AVE 2268; AVE 5688; AVE 8134; TA-6666; AZD 6370; SSR 162369; TLK-17411; NN 2501; MK 431; KGA-2727; MK-767; CS-872; a beta-3 receptor antagonist such as N-5984; an alpha-glucosidase inhibitor such as acarbose, voglibose or miglitol; a glinitide/meglitinide analogue or carbamoylmethylbensoeic acid derivative such as mitiglinide, repaglinide or nateglinide; a DPP-IV inhibitor such as LAF 237 (vildagliptin), DPP728, P93/01, P32/98, PT-630 or saxagliptin; GLP-1 or GLP-1 analogues, such as exenatide, Exenatide-LAR, liraglutide (NN 2211), ZP 10/AVE 0010, LY 307161, betatropin, CJC-1131, GTP-010, SUN E7001 or AZM 134; pramlinitide acetate; insulin or insulin analogues, such as Humalog (insulin lispro), Humulin, Novolin, Novolog/NovoRapid (insulin aspart), Apidra (insulin glulisine), Lantus (insulin glargine), Exubera, Levemir/NN 304 (insulin detemir), AERx/NN 1998, Insuman, Pulmonary insulin or NN 344; sibutramine or other blockers of the presynaptic reuptake of serotonin and noradrenalin; orlistat and other inhibitors of GI lipases; β3-adrenergic receptor agonists; uncoupling proteins; (specific) antagonists of PPARγ (Peroxisome Proliferator-Activated Receptor γ); insulin secretagogues; rimonabant and other CB1 endocannabinoid receptor antagonists; bupropion; topiramate; leptin agonists; ciliary neurotrophic factor; peptide analogues of the human growth hormone fragment 177-191; cholecystokinin-A receptor agonists; melanocortin-3 agonists; noradrenergic drugs such as phentermine, diethylpropion, phendimetrazine or benzphetamine; or any combination of the anti-diabetic or anti-obesity drugs mentioned above, and/or

h) a drug potentially useful in the treatment of substance abuse e.g. alcohol abuse such as naltrexone, acamprosate, disulphiram or Vivitrex (naltrexone long acting injection), and/or,

i) a drug potentially useful in the treatment of Crohn's disease such as

1. 5-ASA compounds such as sulfasalazine, oral 5-ASA formulations or rectal 5-ASA formulations,

2. glucocorticosteroids such as systemic steroids (e.g. budesonide or prednisolone) or topically acting steroids (e.g. budesonide),

3. antibiotics such as metronidazole or quinolones (e.g. ciprofloxacine, ofloxacine, norfloxacine, levofloxacine or moxifloxacine),

4. immunosuppressives such as azathioprine, 6-mercaptopurine or methotrexate,

5. nutritional therapies such as elemental or polymeric formulas or pre- and probiotics,

6. biological therapies e.g. TNF-α inhibitors such as infliximab, adalimumab, CDP870, CDP571, etanercept or onercept,

7. symptomatic agents such as anti-diarrheals or anti-spasmodics.

Examples of suitable NSAIDs are piroxicam, diclofenac, nabumetone, propionic acids including naproxen, flurbiprofen, fenoprofen, ketoprofen and ibuprofen, fenamates including mefenamic acid, paracetamol, indomethacin, sulindac, meloxicam, apazone, pyrazolones including phenylbutazone, salicylates including aspirin.

Examples of suitable COX-2 inhibitors are rofecoxib (Vioxx), valdecoxib (Bextra), celecoxib (Celebrex), etoricoxib (Arcoxia), lumiracoxib (Prexige), parecoxib (Dynastat), deracoxib (Deram), tiracoxib, meloxicam, nimesolide, (1,1-dimethylheptyl)-6a,7,10,10a-tetrahydro-I-hydroxy-6,6dimethyl-6H-dibenzo[b,d]pyran carboxylic acid (CT-3), 2(5H)-Furanone, 5,5-dimethyl (I-methylethoxy) [4(methylsulfonyl)phenyl]- (DFP); Carprofen (RIMADYL), (Acetyloxy)-benzoic acid, 3-[(nitrooxy)methyllphenyl ester (NCX4016), P54 (CAS Reg. No. 130996 0) 2,6-Bis(1,1-dimethylethyl) [(E)-(2-ethyl-1,1-dioxo isothiazolidinylidene)methyl]phenol (S-2474), 5(R)-Thio sulfonamide-3(2H)-benzofuranone (SVT-2016) and N-[3-(Fonnyl-amino) oxo phenoxy-4H benzopyran yl] methanesulfonamide ("T-614"); or a pharmaceutically acceptable salt thereof.

Examples of suitable COX/5-LOX inhibitors are licofelone (ML-3000 or [2,2-dimethyl-6-(4-chlorophenyl)-7-phenyl-2,3,dihydro-1H-pyrrolizine-5-yl]-acetic acid), di-tert-butylphenols, such as (E)-(5)-(3,5-di-tert-butyl-4-hydroxybenzylidence)-2-ethyl-1,2-isothiazolidine-1,1-dioxide (S-2474), darbufelone or tebufelone and pharmacologically active metabolites as well as derivatives such as dihydro-dimethyl-benzofuran and PGV-20229, dihydro-dimethyl-benzofuran, thiophene derived compounds such as RWJ-63556, N-hydroxy-N-methyl-4-(2,3-bis-(4-methoxyphenyl)-thiophen-5-yl)-butanamide (S19812), methoxytetrahydropyran derivatives, oxygenated xanthones such as 1,3,6,7-Tetrahydroxyxanthone (norathyriol) - pyrazole thiocarbamates, pyrazoles such as modified forms of phenidone containing compounds or the tri-flouro-benzole substituted pyrazoline derivative BW-755C, tepoxaline and derivatives and di-tert-butylpyrimidines.

It is contemplated that such combination therapy leads to an improved therapeutic response and/or an increased convenience for the individual, compared to said individual being treated without the compound, composition or kit according to the invention.

In a further aspect, the invention relates to a method of reducing side effects associated with oral treatment of any of the conditions a-j listed above, in which method the active pharmaceutical ingredient for treating said condition is used in combination with one or more of the following agents:

a) an antacid such as 1) magnesium hydroxide, 2) magnesium trisilicate, 3) aluminium hydroxyde gel, 3) sodium hydrogencarbonate, 4) magaldrat or any combination of 1-5 and/or

b) a histamine H-2 antagonist such as 1) cimetidine, 2) ranitidine, 3) nizatidine, 4) famotidine, 5) roxatidine, 6) lafutadine or any combination of 1-6 and/or

c) a cytoprotective agent such as 1) sucralfate, 2) tripotassium dictitratobismuthate, 3) carbenoxolone, 4) prostaglandin E-2 analogues such as misoprostol, 5) ecabet, 6) cetraxate HCl, 7) teprenone, 8) troxipide, 9) dicyclomine hydrochloride, 10) sofalcon or any combination of 1-10 and/or

d) a proton pump inhibitor (PPI) such as 1) omeprazole, 2) esomeprazole, 3) lansoproazole, 4) pantoprazole, 5) rabeprazole, 6) CS-526/R-105266, 7) AZD 0865, 8) soraprazan or any combination of 1-8.

e) an NSAID or a COX or a LOX inhibitor such as e.g. a COX-2 inhibitor or a COX/5-LOX inhibitor, and/or

f) pentoxifylline, e.g. at a dose range of from 400 to 800 mg/day.

In a specific aspect, the compound according to the invention is used in combination with a fumaric acid ester containing compound. In particular the fumaric acid ester containing compound is any and all of the salts contained in Fumaderm® or Fumaraat® or Panaclar® (BG-12) or described in US 6,277,882, US 6,355,676 or US 6,509,376. The compound according to the invention may be provided in a formulation according to the present invention, or any Fumaderm® or Fumaraat® or Panaclar® (BG-12) formulation or as e.g. described in US 6,277,882, US 6,355,676, US 6,509,376 or PCT/DK2005/000648.

*Cosmetic and/or pharmaceutical compositions*

The novel esters of the invention may be presented in the form of a cosmetic or pharmaceutical composition. In a further aspect of the invention, the pharmaceutical composition is in the form of a controlled release composition. In one aspect of the invention, the pharmaceutical composition has an enteric coating.

The term "metabolite" as used herein means any intermediate or product resulting from metabolism. In one aspect of the invention, the metabolite is the ester of alkyl fumarate corresponding to the compound according to the invention.

The term "metabolism" as used herein refers to the biotransformation of an active substance administered to a patient. In one embodiment, such biotransformation comprises enzymatic degradataion, hydrolysis and hydrolysis by esterases.

The esters according to the invention may be used for preparing compositions for oral administration in the form of micro-pellets, micro-tablets, capsules (such as soft and hard gelatine capsules), granulates and tablets such as

e.g. described in US 6,509,376 or US 6,355,676 incorporated herein by reference. Further suitable pharmaceutical preparations are preparations for cutaneous and transdermal administration in the form of ointments, plasters, lotions or shower preparations and for parenteral administration in the form of aqueous micro-dispersions, oil-in-water emulsions or oily solutions for rectal administration of suppositories or micro-enemas.

The novel esters may solve or reduce the problems related to the appearance of gastro-intestinal side-effects and/or flush side-effects upon oral administration of the known fumaric acid esters. Furthermore, by prolonging and/or delaying the release of the active substance and/or metabolites thereof from the composition it is envisaged that the local concentration of the active substance at specific sites of the gastro-intestinal tract is reduced (compared with that of Fumaderm@) which in turn leads to a reduction in gastro-intestinal side-effects and/or flushing. Accordingly, compositions that enable a prolonged and/or a slow release of a compound according to the invention and/or a metabolite thereof are within the scope of the present invention.

Such compositions are well-known to the skilled artisan and include e.g. diffusion-controlled drug delivery systems, osmotic pressure controlled drug delivery systems, erodible drug delivery systems etc. Moreover, there are pharmaceutical companies that based on a specific technology (such as mentioned above) can provide a specific composition with specific release characteristics of the active substance and/or a metabolite thereof. Accordingly, a person skilled in the art will know how to obtain a suitable product once he has realized a specific need in respect of a particular drug substance. By way of example, Eurand is one of such companies that offer technical solutions in order to obtain a controlled release pharmaceutical composition containing a specific active substance and having specific requirements with respect to the release of the active substance from the composition (see e.g. http://www.eurand.com). Another company is MacroMed, Inc. that has developed a technology involving a so-called SQZgel™ (http://macromed.com, SQZgel™'s mechanism of action is a pH-sensitive polymer mixture combined with an outer coating. In the acidic environment of the stomach the polymer imbibes with water and swells, entrapping the drug. Upon entering the higher pH of the intestines, the polymer slowly shrinks, or "squeezes" at a "dialed-in" rate releasing the active composition in a sustained manner.), or Egalet a/s that has a specific extrusion based technology (http://www.egalet.com, Key elements of the Egalet® technology are a biodegradable coat and a matrix, comprising the active drug, which is surface erodible, hydrophobic and composed of PEG-stearate. One of the Egalet® technologies is the 2K Egalet® constant release system, which is a 2-component production model consisting of coat and matrix. The drug is evenly distributed throughout the Egalet® matrix for constant release over time). These and other technologies like e.g. the Eurand technologies Diffucaps (Drug release profiles are created by layering active drug onto a neutral core such as sugar spheres, crystals or granules followed by a rate-controlling, functional membrane. Diffucaps/Surecaps beads are small in size, approximately 1mm or less in diameter. By incorporating beads of differing drug release profiles into hard gelatin capsules, combination release profiles can be achieved.), Diffutabs (The Diffutab technology incorporates a blend of hydrophilic polymers that control drug release through diffusion and erosion of a matrix tablet.), Minitabs (Eurand Minitabs are tiny (2mm x 2mm) tablets containing gel-forming excipients that control drug release rate. Additional membranes may be added to further control release rate.), Orbexa (This technology produces beads that are of controlled size and density with a defined-based granulation extrusion and spheronization techniques. The resultant beads can be coated with release rate controlling membranes for additional release rate control and may be filled into capsules or provided in sachet form.) and SDS (Eurand's SDS technology uses functional polymers or a combination of functional polymers and specific additives, such as composite polymeric materials, to deliver a drug to a site of optimal absorption along the intestinal tract. In order to achieve this, Eurand first produces multiparticulate dosage forms such as Diffucaps or Eurand Minitabs, which incorporate the active drug. These dosage forms are then coated with pH dependent/independent polymeric membranes that will deliver the drug to the desired site. These are then filled into hard gelatin capsules.) are also of interest in the present context.

An interesting technology for use in formulating compositions according to the present invention is the so-called MeltDose® technology as described in WO 03/004001 (see http://www.lifecyclepharma.com. MeltDose® involves formulating solubilized, individual molecules into tablets. By formulating individual molecules, the primary limitation of oral absorption of drugs with low water-solubility is removed, and a superior bioavailability can be attained.). By employing this technology it is possible to obtain a particulate material that is suitable for processing into various pharmaceutical dosage forms e.g. in the form of pellets or tablets. Furthermore, the technology is suitable for use as it is possible to obtain a suitable release profile of the active substance and/or a metabolite thereof, e.g. such as those release profiles described herein. In one embodiment, pellets suitable for use may have a mean particle size larger than 2000 μm. In another embodiment, pellets suitable for use may have a mean particle size of from about 0.01μm to about 250 μm.

Another specific suitable formulation principle for use in the present context is formulation in a lipophilic environment such as, e.g., soft gelatin capsules. Vegicaps Soft from Scherer (a soft capsule technology based on carrageenan and starch. While this new dosage form is 100% plant-derived, it still offers all the key attributes of traditional soft gelatin capsules. These include a soft and flexible dosage form that provides ease of swallowing.) is a suitable example of such a formulation principle (please refer to http://www.rpscherer.de/page.php?pageID=94).

A further specific example of a suitable formulation comprises the compound according to the invention together

with vitamin E concentrate in soft or hard gelatin capsules. This formulation, in a modified form, is the basis of the commercial cyclosporine product, Neoral®, containing, among other things, corn oil-mono-di-triglycerides, polyoxyl 40 hydrogenated castor oil NF, DL-α-tocopherol USP (part of the vitamin E family), gelatin NF, glycerol, iron oxide black, propylene glycol USP, titanium dioxide USP, carmine, and alcohol in addition to cyclosporine.

Another specific example of a suitable formulation comprises the compound according to the invention together with ethanol, tocopherolethylene glycol 1000 succinate (TPGS), corn oil and wax in soft or hard gelatin capsules. This product can be a semi-solid or solid dosage form. The release rate of this formulation is dependent on erosion due to lipases in the intestine.

A further example of a suitable formulation comprises the formulation of a compound according to the invention together with ethanol, tocopherolethylene glycol 1000 succinate (TPGS), corn oil and polyglycolized glycerides (e.g. Gelucire) in soft or hard gelatin capsules. This product can be a semi-solid or solid dosage form. The release rate of this formulation is dependent on degradation due to lipases in the intestine.

A further example of a suitable formulation is an oral pulsed dose drug delivery system. This dosage form can be perceived as a modified form of the Schering Repetab tablets. A portion of the composition of the present invention is put in the core of a tablet.

The core can for example be made by conventional wet granulation or continuous granulation such as extrusion followed by compaction of the granulate into tablets. The core is then coated using an appropriate technology, preferably by airsuspension using an enteric coating polymer such as Eudragits.

The first releasing dose is compression coated on the core or air-suspension coated either with the enteric coat or on top of the enteric coat. In an embodiment of the invention, the first releasing dose is air-suspension coated with the enteric coat. In a further embodiment of the invention, the first releasing dose is compression coated on the core, in order to avoid release of the composition according to the invention prior to the degradation of the enteric coat, such degradation typically occurring at pH values higher than those found in the gastric ventricle; i.e. the degradation of the enteric coat typically occurs after passage of the gastric ventricle.

A further example of a suitable formulation is an oral sustained drug delivery system. A portion of the composition of the present invention is put in the core of a tablet.

The core can for example be made by conventional wet granulation or continuous granulation such as extrusion followed by compaction of the granulate into tablets. The core is coated using an appropriate technology, preferably by airsuspension using ethylcellulose and a hydrophilic excipient such as hydroxyl propyl cellulose (HPC).

The first releasing dose is compression coated on the core or air-suspension coated either with the enteric coat or on top of the enteric coat. In a preferred embodiment of the invention, the first releasing dose is air-suspension coated with the enteric coat. In a further embodiment of the invention, the first releasing dose is compression coated on the core, in order to avoid release of the composition according to the invention prior to the degradation of the enteric coat, such degradation typically occurring at pH values higher than those found in the gastric ventricle; i.e. the degradation of the enteric coat typically occurs after passage of the gastric ventricle.

A further example of a suitable formulation is obtained via crystal engineering, such as e.g. described in WO 03/080034, which is hereby incorporated by reference.

Accordingly, in another embodiment the composition of the invention comprises the novel ester in the form of micro-crystals with hydrophilic surfaces. Furthermore, in another embodiment of the invention, the micro-crystals are filmcoated directly, in order to achieve a sustained release formulation.

Another specific example of a suitable formulation comprises complexation of the compound according to the present invention with genuine cyclodextrins and cyclodextrin-derivatives (e.g. alkyl- and hydroxyalkyl-derivatives or sulfobutyl-derivatives). The complexation is achieved in accordance with well known methods. It is contemplated that such a complexation leads to a higher solubility and a higher dissolution rate of the composition according to the invention, compared to the composition prior to complexation. Furthermore, it is contemplated that such a complexation leads to a higher bioavailability of the compound according to the invention and/or a metabolite thereof, compared to the composition prior to complexation.

In specific embodiments, the invention relates to a controlled release pharmaceutical composition that may be administered one, two or more times daily, such as once, twice or three times daily. Furthermore, the composition may be designed so that it releases the compound according to the invention and/or a metabolite thereof relatively independent of pH, i.e. the release is not dependent on pH in the gastrointestinal tract. Examples of such compositions are e.g. compositions in the form of solid dosages forms (e.g. tablets, capsules, pellets, beads etc.) that are coated with a controlled release coating. Suitable materials for controlled release coatings are e.g. cellulose and cellulose derivatives including methylcellulose, ethylcellulose and cellulose acetate, or poly(ethylene-co-vinyl acetate), poly (vinyl chloride).

The release of the compound according to the invention and/or a metabolite thereof typically takes place in three steps from a composition coated with a diffusion controlled membrane:

i) firstly, water (from the GI tract) diffuses into the dosage form from the surroundings,

ii) secondly, at least some of the compound according to the invention present in the dosage form dissolves by the action of water,

iii) the dissolved compound according to the invention and/or a metabolite thereof diffuses out of the dosage form and into the surroundings (i.e. the GI tract)

Other examples include e.g. matrix tablets or dosage form containing a multiplicity of units each in the form of a matrix system. The active substance is embedded in a matrix containing e.g. cellulose and cellulose derivatives including microcrystalline cellulose, hydroxypropyl methyl cellulose, hydroxypropyl cellulose and methylcellulose, povidone, poly(ethyleneoxide) (PEO), polyethylene glycol (PEG), poly (vinyl alcohol) (PVA), xanthan gum, carrageenan and other synthetic materials. Substances normally used as pharmaceutically acceptable excipients or additives may be added to a matrix composition.

Other examples of suitable compositions are e.g. hydrogels, i.e. monolithic systems wherein the active substance is embedded in a water-swellable network polymer. Materials suitable for use include e.g. hydrophilic vinyl and acrylic polymers, polysaccharides like alginates, and poly(ethylene oxide).

In specific embodiments, a composition according to the invention has a pH controlled release (also known as pH dependent release) of the compound according to the invention and/or a metabolite thereof. Normally, the release is designed so that only a small amount, if any, of the compound according to the invention and/or a metabolite thereof is released in the stomach (pH up to about 3), whereas the compound according to the invention and/or a metabolite thereof is released in the intestines (pH shifts to about 6-7). Such a pH controlled release can be obtained by providing a composition of the invention with an enteric coating (the whole composition or, if the composition is a multiparticulate composition, the individual units) or by providing a composition that releases the compound according to the invention and/or a metabolite thereof by a pH-dependent osmotic mechanism, or by employment of suitable enzymes.

Examples of suitable substances for use as enteric coating materials include polyacrylamides, phthalate derivatives such as acid phthalates of carbohydrates, amylose acetate phthalate, cellulose acetate phthalate, other cellulose ester phthalates, cellulose ether phthalates, hydroxypropylcellulose phthalate, hydroxypropylethylcellulose phthalate, hydroxypropylmethylcellulose phthalate, methylcellulose phthalate, polyvinyl acetate phthalate, poly acrylic methacrylic acid copolymers, shellac and vinyl acetate and crotonic acid copolymers, etc.

The compositions mentioned above having a pH independent release may also be formulated to release the compound according to the invention and/or a metabolite thereof e.g. by providing the composition with an outer layer of an enteric coating.

Furthermore, the compositions may be formulated in such a manner that an initial delay in release of the compound according to the invention and/or a metabolite thereof is obtained. Such a delay may be obtained e.g. by choosing an outermost coating that in a time-controlled manner degrades (e.g. erodes) and only when this outermost coating is eroded away, the release of the compound according to the invention and/or a metabolite thereof starts.

In one aspect of the invention, the compound according to the invention is formulated in a composition that enables a prolonged and/or a slow release of a compound according to the invention and/or a metabolite thereof as defined above. Examples of such compositions are for example described in PCT/DK2005/000648 which is hereby incorporated by reference.

In the present context, a controlled release composition is a composition that is designed to release the compound according to the invention and/or a metabolite thereof in a prolonged, slow and/or delayed manner compared to the release of the commercially available product Fumaderm@, when tested under comparable conditions (e.g. for *in vivo* studies: dose equivalents, with or without standardized meal etc., or for *in vitro* studies: dose equivalents, dissolution test apparatus and working conditions including e.g. composition, volume and temperature of dissolution medium employed, rotation speed etc.).

The release *in vivo* may be tested by measuring the plasma concentration at predetermined time periods and thereby obtaining a plasma concentration versus time profile for the compound according to the invention in question or, if relevant, a metabolite thereof. Furthermore, it is contemplated that metabolism already takes place within the gastro-intestinal tract or during passage of the gastro-intestinal mucosa, or upon first passage through the hepatic circulation. Other tests may also be used to determine or to give a measure of the release of the active substance *in vivo*. Thus, animals (e.g. mice, rats, dogs etc.) may be used as a model. The animals receive the compositions under investigation and after specified periods of time, the animals are sacrificed and the content of the active ingredient (or metabolite thereof, if relevant) is determined in plasma or specific organs or extracted from the intestinal contents.

Another test involves the use of a specific segment of an animal intestine. The segment is placed in a suitable dissolution apparatus containing two compartments (a donor and a receiver) separated by the segment, and the composition under investigation is placed in a suitable medium in one compartment (the donor compartment). The composition will release the compound according to the invention and/or a metabolite thereof that subsequently is transported across the intestinal segment. Accordingly, at suitable time intervals the concentration of the active substance (or, if relevant,

the metabolite) is measured in the receiver compartment.

A person skilled in the art will be able to adapt the above-mentioned method to the specific composition.

With respect to *in vitro* methods, well-established methods are available, especially methods described by official monographs like e.g. United States Pharmacopeia (USP) or the European Pharmacopoeia. A person skilled in the art will know which method to choose and how to select the specific conditions to carry out the *in vitro* test. For instance, the USP prescribes *in vitro* tests be carried out at 37 +/- 1.0 such as 37 +/-0.5 degrees Celsius/Centigrade. A suitable dissolution test is, for example for capsules, wherein the dissolution profile is determined as described in the United States Pharmacopoeia at 37˚C using a rotating basket at 100 rpm employing 0.1 N hydrochloric acid as dissolution medium during the first 2 hours of the test and then followed by 0.05 M phosphate buffer pH 6.5 as dissolution medium for the remaining test period, and, for example as described for tablets wherein the dissolution profile is determined as described in the United States Pharmacopoeia at 37˚C using a paddle dissolution apparatus at 100 rpm employing 0.1 N hydrochloric acid as dissolution medium during the first 2 hours of the test and then followed by 0.05 M phosphate buffer pH 6.5 as dissolution medium for the remaining test period.

As mentioned above, the in vivo release of the compound according to the invention and/or a metabolite thereof is in one aspect of the invention prolonged, slow and/or delayed compared with the commercially available Fumaderm@ composition.

With regard to the compound according to the invention, the term "prolonged" is in one embodiment intended to indicate that the active substance is released during a longer time period than Fumaderm@ such as at least during a time period that is at least 1.2 times, such as, e.g., at least 1.5 times, at least 2 times, at least 3 times, at least 4 times or at least 5 times greater than that of Fumaderm@. Thus, if e.g. 100% of dimethylfumarate is released from Fumaderm@ tablets 3 hours after the start of a suitable test, then 100% of the compound according to the invention and/or a metabolite thereof in a composition according to the invention is released at least 3.6 hours after the start of a suitable test.

With regard to the compound according to the invention and/or a metabolite thereof the term "delayed" is in one embodiment intended to indicate that the release starts at a later point in time compared with that of Fumaderm® (such as at 30 min or more later such as, e.g., 45 min or more later, 1 hour or more later or 1.5 hours or more later, alternatively, that the initial release during the first 2 hours is much less compared with that of Fumaderm@ (i.e. less than 80% w/w such as, e.g., less than 70% w/w, less than 60% w/w or less than 50% of that of Fumaderm®).

As a particular useful composition comprising the compound according to the invention is a controlled release composition designed to be administered two or more times daily, such as e.g. described in PCT/DK2005/000648 which is hereby incorporated by reference.

In the following is given a description of various compositions according to the invention that are designed to in one embodiment obtain a suitable release of the compound according to the invention and/or a metabolite thereof. Based on the description above and handbooks within the field of controlled release of pharmaceutics, a person skilled in the art will know how to choose different formulation principles in order to achieve the required release profile.

*Compositions designed to be administered two or more times daily*

*pH dependent release*

In the following is given a description of specific embodiments, wherein the compound according to the invention and/or a metabolite thereof is released depending on pH and wherein the release pattern is suitable for compositions that are administered two or more times daily. Examples of suitable formulation principles are e.g. compositions provided with an enteric coating or hydrogels of a type described by Zentner et al (US 6,537,584) and Bae (US 5,484,610), which hereby are incorporated by reference. Further examples of suitable formulation principles are e.g. compositions provided with a diffusion coating such as a controlled release diffusion coating, matrix particulates or matrix tablets, hydrogels, pulsed dose drug delivery systems, co-formulation with vitamin E concentrate or ethanol, TPGS, corn oil and wax etc., including any of the formulation principles mentioned above, optionally with an enteric coating.

Accordingly, in one aspect the invention relates to a controlled release pharmaceutical composition for oral use comprising as an active substance a monosaccharideester of $C_{1-5}$alkyl-fumarate, wherein the release of the compound according to the invention and/or a metabolite thereof - when subjected to an in vitro dissolution test employing 0.1 N hydrochloric acid as dissolution medium during the first 2 hours of the test and then 0.05 M phosphate buffer pH 6.5 or 6.8 as dissolution medium - is as follows:

within the first 2 hours after start of the test at the most about 15% w/w such as, e.g. at the most about 10% w/w, at the most about 5% w/w of the total amount of the compound according to the invention and/or a metabolite thereof is released, and/or

within the first 2 hours after start of the test at least about 1% w/w such as, e.g. at least about 2% w/w, at least about

3% w/w, or about 5% w/w of the total amount of the compound according to the invention and/or a metabolite thereof is released, and/or

within the first 3 hours after start of the test at the most about 35% w/w such as, e.g., from about 15% to about 35% w/w, from about 20% to about 30% w/w, or about 25% w/w of the total amount of the compound according to the invention and/or a metabolite thereof is released, and/or

within the first 3 hours after start of the test at the most about 90% w/w such as, e.g., from about 5% to about 90% w/w, from about 5% to about 85% w/w, from about 10% to about 80% w/w, from about 10% to about 70% w/w, from about 10% to about 65% w/w, from about 10% to about 60% w/w, from about 15% to about 50% w/w, from about 15% to about 35% w/w, from about 20% to about 30% w/w, or about 20% w/w, or about 25% w/w of the total amount of the compound according to the invention and/or a metabolite thereof is released, and/or

within the first 4 hours after start of the test at the most about 92% w/w such as, e.g., from about 10% to about 92% w/w, from about 20% to about 85% w/w, from about 20% to about 80% w/w, from about 20% to about 70% w/w, from about 25% to about 60% w/w, from about 25% to about 55% w/w, from about 30% to about 50% w/w, or about 35% w/w, or about 40% w/w, or about 45% w/w of the total amount of the compound according to the invention and/or a metabolite thereof is released, and/or

within the first 5 hours after start of the test at the most about 94% w/w such as, e.g., from about 15% to about 94% w/w, from about 25% to about 90% w/w, from about 30% to about 85% w/w, from about 35% to about 80% w/w, from about 35% to about 75% w/w, from about 40% to about 70% w/w, from about 45% to about 70% w/w, from about 55% to about 70% w/w, from about 60% to about 70% w/w, or about 45% w/w, or about 50% w/w, or about 55% w/w, or about 60% w/w, or about 65% w/w of the total amount of the compound according to the invention and/or a metabolite thereof is released, and/or

within the first 6 hours after start of the test at the most about 60% w/w such as, e.g., from about 30% to about 60% w/w, from about 40% to about 55% w/w, or about 50% w/w of the total amount of the compound according to the invention contained in the composition and/or a metabolite thereof is released, and/or

within the first 6 hours after start of the test at the most about 95% w/w such as, e.g., from about 35% to about 95% w/w, from about 40% to about 90% w/w, from about 45% to about 85% w/w, from about 50% to about 85% w/w, from about 55% to about 85% w/w, from about 60% to about 85% w/w, from about 65% to about 85% w/w, from about 70% to about 85% w/w, from about 75% to about 85% w/w, or about 65% w/w, or about 70% w/w, or about 75% w/w, or about 80% w/w of the total amount of the compound according to the invention contained in the composition and/or a metabolite thereof is released, and/or

within the first 7 hours after start of the test at the most about 98% w/w such as, e.g., from about 45% to about 98% w/w, from about 50% to about 98% w/w, from about 55% to about 98% w/w, from about 60% to about 98% w/w, from about 65% to about 98% w/w, from about 70% to about 98% w/w, from about 75% to about 95% w/w, from about 80% to about 95% w/w, from about 85% to about 95% w/w, or about 75% w/w, or about 80% w/w, or about 85% w/w, or about 90% w/w of the total amount of the compound according to the invention contained in the composition and/or a metabolite thereof is released, and/or

within the first 9 hours after start of the test at the most about 85% w/w such as, e.g., from about 50% to about 85% w/w, from about 60% to about 80% w/w, or about 75% w/w of the total amount of the compound according to the invention contained in the composition and/or a metabolite thereof is released, and/or

within the first 9 hours after start of the test at the most about 99% w/w such as, e.g., from about 60% to about 99% w/w, from about 70% to about 99% w/w, from about 80% to about 99% w/w, from about 90% to about 99% w/w, or about 95% w/w of the total amount of the compound according to the invention contained in the composition and/or a metabolite thereof is released, and/or

within the first 12 hours after start of the test at least about 80% w/w such as, e.g., about 80% w/w or more, about 85% w/w or more, about 90% w/w or more or about 95% w/w or more of the total amount of the compound according to the invention contained in the composition and/or a metabolite thereof is released.

*Compositions designed to be administered once daily*

*pH dependent release*

In the following is given a description of specific embodiments, wherein the compound according to the invention and/or a metabolite thereof is released dependently of pH and wherein the release pattern is suitable for compositions that are administered once daily. Examples of suitable formulation principles are e.g. compositions provided with an enteric coating or hydrogels of a type described by Zentner et al (US 6,537,584) and Bae (US 5,484,610). Further examples of suitable formulation principles are e.g. compositions provided with a diffusion coating such as a controlled release diffusion coating, matrix particulates or matrix tablets, hydrogels, pulsed dose drug delivery systems, co-formulation with vitamin E concentrate or ethanol, TPGS, corn oil and wax etc., including any of the formulation principles mentioned above, optionally with an enteric coating.

Accordingly, in one aspect the invention relates to a controlled release pharmaceutical composition for oral use comprising as an active substance a monosaccharideesters of $C_{1-5}$alkyl- fumarate, wherein the release of the compound according to the invention and/or a metabolite thereof - when subjected to an in vitro dissolution test employing 0.1 N hydrochloric acid as dissolution medium during the first 2 hours of the test and then 0.05 M phosphate buffer pH 6.5 or 6.8 as dissolution medium - is as follows:

within the first 2 hours after start of the test at the most about 15% w/w such as, e.g., at the most about 10% w/w or at the most about 5% w/w of the total amount of the compound according to the invention and/or a metabolite thereof is released, and/or

within the first 2 hours after start of the test at least about 1% w/w such as, e.g. at least about 2% w/w, at least about 3% w/w, or about 5% w/w of the total amount of the compound according to the invention and/or a metabolite thereof is released, and/or

within the first 4 hours after start of the test at the most about 90% w/w such as, e.g., from about 5% to about 90% w/w, from about 5% to about 85% w/w, from about 10% to about 80% w/w, from about 10% to about 70% w/w, from about 10% to about 65% w/w, from about 10% to about 60% w/w, from about 15% to about 50% w/w, from about 15% to about 35% w/w, from about 20% to about 30% w/w, or about 20% w/w, or about 25% w/w of the total amount of the compound according to the invention and/or a metabolite thereof is released, and/or

within the first 4.5 hours after start of the test at the most about 35% w/w such as, e.g., from about 15% to about 35% w/w, from about 20% to about 30% w/w, or about 25% w/w of the total amount of the compound according to the invention and/or a metabolite thereof is released, and/or

within the first 5 hours after start of the test at the most about 92% w/w such as, e.g., from about 10% to about 92% w/w, from about 20% to about 85% w/w, from about 20% to about 80% w/w, from about 20% to about 70% w/w, from about 25% to about 60% w/w, from about 25% to about 55% w/w, from about 30% to about 50% w/w, or about 35% w/w, or about 40% w/w, or about 45% w/w of the total amount of the compound according to the invention and/or a metabolite thereof is released, and/or

within the first 6 hours after start of the test at the most about 94% w/w such as, e.g., from about 15% to about 94% w/w, from about 25% to about 90% w/w, from about 30% to about 85% w/w, from about 35% to about 80% w/w, from about 35% to about 75% w/w, from about 40% to about 70% w/w, from about 45% to about 70% w/w, from about 55% to about 70% w/w, from about 60% to about 70% w/w, or about 45% w/w, or about 50% w/w, or about 55% w/w, or about 60% w/w, or about 65% w/w of the total amount of the compound according to the invention and/or a metabolite thereof is released, and/or

within the first 7 hours after start of the test at the most about 95% w/w such as, e.g., from about 35% to about 95% w/w, from about 40% to about 90% w/w, from about 45% to about 85% w/w, from about 50% to about 85% w/w, from about 55% to about 85% w/w, from about 60% to about 85% w/w, from about 65% to about 85% w/w, from about 70% to about 85% w/w, from about 75% to about 85% w/w, or about 65% w/w, or about 70% w/w, or about 75% w/w, or about 80% w/w of the total amount of the compound according to the invention contained in the composition and/or a metabolite thereof is released, and/or

within the first 9 hours after start of the test at the most about 98% w/w such as, e.g., from about 45% to about 98% w/w, from about 50% to about 98% w/w, from about 55% to about 98% w/w, from about 60% to about 98% w/w,

from about 65% to about 98% w/w, from about 70% to about 98% w/w, from about 75% to about 95% w/w, from about 80% to about 95% w/w, from about 85% to about 95% w/w, or about 75% w/w, or about 80% w/w, or about 85% w/w, or about 90% w/w of the total amount of the compound according to the invention contained in the composition and/or a metabolite thereof is released, and/or

within the first 9 hours after start of the test at the most about 60% w/w such as, e.g., from about 30% to about 60% w/w, from about 40% to about 55% w/w, or about 50% w/w of the total amount of the compound according to the invention contained in the composition and/or a metabolite thereof is released, and/or

within the first 12 hours after start of the test at the most about 99% w/w such as, e.g., from about 60% to about 99% w/w, from about 70% to about 99% w/w, from about 80% to about 99% w/w, from about 90% to about 99% w/w, or about 95% w/w of the total amount of the compound according to the invention contained in the composition and/or a metabolite thereof is released, and/or

within the first 13.5 hours after start of the test at the most about 85% w/w such as, e.g., from about 50% to about 85% w/w, from about 60% to about 80% w/w, or about 75% w/w of the total amount of the compound according to the invention contained in the composition and/or a metabolite thereof is released, and/or

within the first 18 hours after start of the test at least about 80% w/w such as, e.g., about 80% w/w or more, about 85% w/w or more, about 90% w/w or more or about 95% w/w or more of the total amount of the compound according to the invention contained in the composition and/or a metabolite thereof is released, and/or

the total amount of the compound according to the invention contained in the composition and/or a metabolite thereof is released within the first 18 hours after start of the test.

Typically, as described above, the compositions according to the invention are designed to deliver the active substance and/or a metabolite thereof, which in turn is metabolised to fumaric acid and, which subsequently is subjected to a rapid elimination process) in a prolonged manner. Apart from the characteristic *in vitro* release patterns described herein, such a prolonged release is reflected in the pharmacokinetic parameters obtained after a clinical study as well. Accordingly, it is contemplated that the $c_{max}$ of the compound according to the invention and/or a metabolite thereof (which is contemplated to appear in the plasma upon hydrolysis or metabolism of the compound according to the invention) is of the same order of magnitude as previously described in the literature provided that similar or equivalent dose is administered (i.e. e.g. a $c_{max}$ of monomethylfumarate in a range of from about 0.4 to about 2.0 mg/l). However, in order to avoid many frequent daily administrations (2-4 tablets 1-3 times daily) it is an aim to prolong the time period where the concentration is within the therapeutic window. Accordingly, it is contemplated that $W_{50}$ (i.e. the time period in which the plasma concentration is 50% of $c_{max}$ or more) is prolonged compared to the marketed treatment with at least 10% such as, e.g. at least 20%, at least 30%, at least 40% or at least 50%. A suitable $W_{50}$ is believed to be at least 2 hours such as in a range of from about 2 to about 15 hours or from about 2.5 to about 10 hours or from about 3 to about 8 hours.

Furthermore, it is contemplated that a controlled release composition according to the invention may lead to a reduced interindividual and/or intraindividual variation in the plasma profile and to a reduced dependency on whether the composition is taken together with or without food (a reduced variation of the plasma concentration profile of the compound according to the invention and/or a metabolite thereof when the pharmaceutical composition is administered with or without concomitant food intake), compared to e.g. Fumaderm® or compared to the composition of the invention in an immediate release form. Therefore, the controlled release composition according to the invention may lead to a reduced frequency of dosing and/or a reduced average total daily dose.

It is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims. Where a range of values is provided, it is understood that each intervening value, to the tenth of the unit of the lower limit unless the context clearly dictates otherwise, between the upper and lower limit of that range and any other stated or intervening value in that stated range is encompassed within the invention. The upper and lower limits of these smaller ranges may independently be included in the smaller ranges and are encompassed within the invention, subject to any specifically excluded limit in the stated range. Where the stated range includes one or both of the limits, ranges excluding either or both of those included limits are also included in the invention. Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and

materials are described. All publications mentioned herein are incorporated herein by reference to disclose and describe the methods and/or materials in connection with which the publications are cited. It must be noted that as used herein and in the appended claims, the singular forms "a", "an", and "the" include plural referents unless the context clearly dictates otherwise. The patents and publications discussed herein are provided solely for their disclosure prior to the filing date of the present application. Nothing herein is to be construed as an admission that the present invention is not entitled to antedate such patent or publication by virtue of prior invention. Further, the dates of publication provided may be different from the actual publication dates which may need to be independently confirmed. As will be apparent to those of skill in the art upon reading this disclosure, each of the individual embodiments described and illustrated herein has discrete components and features which may be readily separated from or combined with the features of any of the other several embodiments without departing from the scope or spirit of the present invention. The figures shown herein are not necessarily drawn to scale, with some components and features being exaggerated for clarity.

Although the foregoing invention has been described in some detail by way of illustration and example for purposes of clarity of understanding, it is readily apparent to those of ordinary skill in the art in light of the teachings of this invention that certain changes and modifications may be made thereto without departing from the spirit or scope of the appended claims.

## EXAMPLES

### Example 1

Synthesis of D-Galactopyranose, 6-(methyl fumarate) (methyl ((3R,4S,5R,6S)-3,4,5,6-tetrahydroxytetrahydro-2H-pyran-2-yl)methyl fumarate)

Materials:

| Product | Company | Product nr. |
|---|---|---|
| Dichloromethane | Sigma-Aldrich Denmark A/S | 270997 |
| Ethyl acetate | Sigma-Aldrich Denmark A/S | 45780 |
| 1,2:3,4-di-O-isopropylidene-alpha-D-galactopyranose | Sigma-Aldrich Denmark A/S | 38406 |
| Mono-methyl fumarate | Sigma-Aldrich Denmark A/S | 651419 |
| Thionyl chloride | Sigma-Aldrich Denmark A/S | 88950 |
| Triethylamine | Sigma-Aldrich Denmark A/S | 90340 |
| n-Hexane | Sigma-Aldrich Denmark A/S | 34859 |
| NaCl | Merck | 1.06404.1000 |
| $MgSO_4$ | Riedel-de-Häen | 31420 |
| $Al_2O_3$ puris | M&B | |
| Soda lime | Bie & Berntsen | 33109 |
| Silica gel, davisil, grade 634, 100-200 mech, 60 Å | Sigma-Aldrich Denmark A/S | 236780 |
| Aldrich calibrated chromatography column | Sigma-Aldrich Denmark A/S | Z560421 |
| Silica gel/ TLC-cards | Sigma-Aldrich Denmark A/S | 60805 |
| Phosphomolybdic acid hydrate | Sigma-Aldrich Denmark A/S | 79563 |
| Trifluoracetic acid | Sigma-Aldrich Denmark A/S | T62200 |
| di-Phosphorpentoxid | Merck | 570 |
| Octadecyl-functionalized silica gel | Sigma-Aldrich Denmark A/S | 377635 |
| Methanol | Sigma-Aldrich Denmark A/S | 34983 |

Synthesis of methyl fumaroyl chloride:

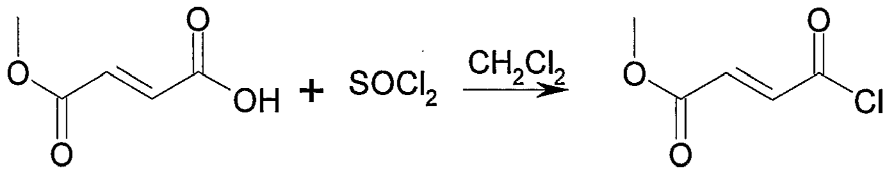


All glass equipment was dried at 110˚C overnight.

3.5582 g monomethyl fumarate was suspended in 40ml dichloromethane and placed in a 250 mL two-necked round bottomed flask, equipped with an addition funnel, vigreux distilling column and thermometer. The solution was heated to 40 ˚C.

7.8 ml thionyl chloride was slowly added (~1 hour) through an addition funnel. Gas formation was observed.

Dichloromethane was slowly distilled at 40 ˚C (oil bath ~55˚C)

The mixture was refluxed at 60-65 ˚C until gas formation stops and no solids were observed in the round bottle. The mixture was slightly yellow.

Excess thionyl chloride was distilled off under reduced pressure (bp: 41˚C/ house vacuum)

Methyl fumaroyl chloride was distilled off at reduced pressure at about 32 ˚C/ oil pump.
Yield: 1.61g
Yield%: 61*100/ 4.06 = 39.63%
Mp.: 12-13˚C

Calculations

| | Monomethyl fumarate | $SOCl_2$ | Methyl fumaroyl chloride | HCl | $SO_2$ |
|---|---|---|---|---|---|
| Mw | 130.10 | 118.97 | 148.54 | 36.46 | 64.06 |
| m | 3.5582 | 7.8ml | 4.06 | 1.00 | 1.75 |
| n | 0.02735 | 0.1094 | 0.02735 | 0.027 | 0.027 |

Synthesis of 1,2:3,4-di-O-isopropylidene α- D-galactopyranose,6-(methyl fumarate) (methyl ((3aR,5R,5aS,8aS,8bR)-2,2,7,7-tetramethyltetrahydro-3aH-bis[1,3]dioxolo[4,5-b:4',5'-d]pyran-5-yl)methyl fumarate)

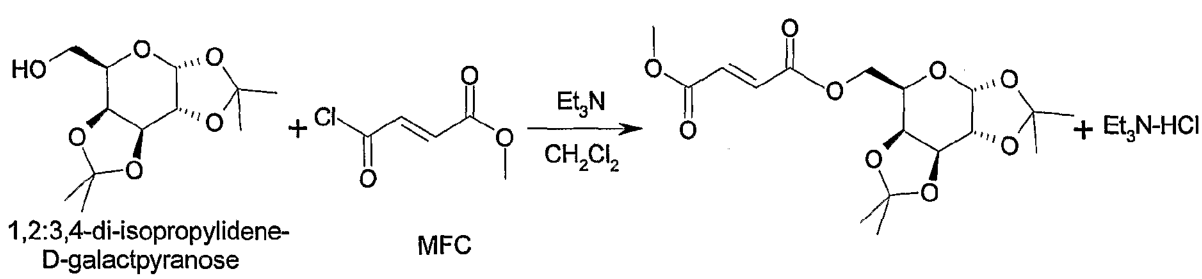


1,2:3,4-di-isopropylidene-D-galactpyranose

MFC

1.61g methyl fumaroyl chloride was dissolved in 100 ml dichloromethane in a 500 ml two-necked round-bottomed flask equipped with an addition funnel and a condenser with a drying tube at the top containing soda lime.

The mixture was cooled in an ice/water bath, where upon 1.23ml triethylamine was added.

2.29g 1,2:3,4-di-o-isopropylidene α- D-galactopyranose was dissolved in 20 ml dichloromethane and slowly added to the mixture through an addition funnel.

The reaction was continued for 24 hours in an ice/water bath which eventually reached room temperature.

Dichloromethane was removed by rotary evaporation.

The crude product was dissolved in 100 ml ethyl acetate

Triethylamine-HCl was removed by suction filtration. Mp(TEA-HCl): 250-253 °C

The organic phase was extracted with 3* 50 ml dem. water and 1* 60 ml saturated NaCl/water

The organic phase was dried with $MgSO_4$ overnight

$MgSO_4$ was removed by suction filtration

20g $Al_2O_3$ was added to the mixture and stirred with heating for 45min.

$Al_2O_3$ was removed by suction filtration

Ethyl acetate was removed by rotary evaporation. The crude product formed crystals on standing at room temperature.

TLC: two spots were observed using Ethyl acetate/ n-hexane 1:1.

○ $Rf_1$:0.88 - identified by NMR as 1,2:3,4-di-o-isopropylidene-alpha- D-galactopyranose,6-(methyl fumarate)

○ $Rf_2$: 0.51 - same Rf as 1,2:3,4-di-o-isopropylidene-alpha- D-galactopyranose, and was properly 1,2:3,4-di-o-isopropylidene-alpha- D-galactopyranose.

The crude product was dissolved in 2ml ethyl acetate and ran through a column containing ~300g silica gel. Mobile phase: Ethyl acetate/ n-hexane 1:1(v/v). Abs. detection at 255nm.

○ The fractions containing 1,2:3,4-di-o-isopropylidene-alpha- D-galactopyranose,6-(methyl fumarate) were pooled.

○ The mobile phase was removed by rotary evaporation and 1,2:3,4-di-o-isopropylidene-alpha- D-galactopyranose, 6-(methyl fumarate) was collected as white crystals

Yield: 50%

*Calculation*

| | DIPG DIPG/MMF: 0.8 | Methyl fumaroyl chloride | Triethylamine (d: 0.726 g/ml) | DIPG-MMF | Triethylamin*H Cl |
|---|---|---|---|---|---|
| Mw | 260.29 | 148.54 | 101.19 | 372.37 | 137.65 |
| m | 2.29g | 1.61 | 1.23ml | 3.28 | 1.21 |
| n | 0.0088 | 0.0108 | 0.0088 | 0.0088 | 0.0088 |

Removal of isopropylidene groups - Synthesis of D-Galactopyranose, 6-(methyl fumarate)

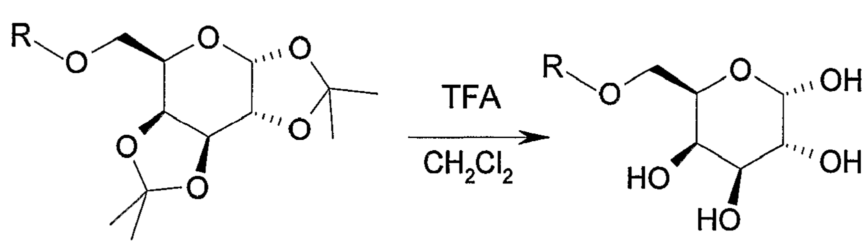

0.7542g 1,2:3,4-di-O-isopropylidene-alpha- D-galactopyranose,6-(methyl fumarate) was dissolved in 7.5ml dichloromethane, after which 7.5ml triflouroacetic acid was added.

The mixture was stirred at room temperature for 20 hours.

The solvent was removed by rotary evaporation.

2*20ml dem. water was added to the solution and evaporated by rotary evaporation.

The remaining product was dried in a dessicator with vacuum and $P_2O_5$.

A brownish solid remains.

m(crude product): 0.63g

The crude product was dissolved in 4ml methanol and added to a column containing 20g octadecyl-functionalized silica gel. Mobile phase methanol/ water 1:1(v/v). Abs. detection at 255nm.

- ○ The fractions containing D-galactopyranose,6-(methyl fumarate) were pooled.
- ○ The mobile phase was removed by rotary evaporation and D-galactopyranose,6-(methyl fumarate) was collected.

Yield: 0.49g
Yield%: 82.8%

| | DIPG-MMF | TFA | Galactose-MMF | Acetone |
|---|---|---|---|---|
| Mw | 372.37 | 114.02 | 292.25 | 58 |
| m | 0.7542 | 10.36 | 0.59 | 0.23 |
| n | 0.0020 | 0.0909 | 0.0020 | 0.0041 |

**Example 2**

**Measuring intestinal permeability using Caco-2 cell monolayers.**

MATERIALS AND METHODS

Cell Culture:

Caco-2 cells are cultured in Dulbecco's modified Eagle's medium (DMEM), containing 10% fetal bovine serum (FBS), 1 mM sodium pyruvate, 100 μM non-essential amino acids, 2 mM L-glutamine, 100 U/mL penicillin, and 100 μg/mL streptomycin. Cells are cultured in 175 $cm^2$ flasks (Costar USA). For transport studies, cells are harvested from the flasks with a trypsin-EDTA (0.25% (w/v)-1 mM EDTA) solution (Gibco Life Technologies) and are seeded at a density of 60,000 cells/$cm^2$ on collagen-coated Transwell ® polycarbonate filters (0.4 μm pore size, 1.13 $cm^2$ surface area) (Costar 3401). Cells on flasks or Transwell® are cultured at 37˚C in a humidified atmosphere containing 5%$CO_2$. Culture media is changed every other day for 10 days, and daily afterwards. Cell monolayers are used between 21 and 28 days post-seeding.

Permeability Assay:

The permeability assay buffer (PAB) is Hank's balanced salts solution containing 15 mM D(+)glucose and 10 mM HEPES, pH 7.3 ± 0.1 The quality control of cell monolayers to be used in the permeability assays is conducted in two steps. The first step consists of certifying the suitability of the batch of cells and the second step consist of testing each monolayer in the entire seeding. To certify a batch (seeding) of cells, a subgroup of randomly-selected monolayers is tested with respect to transepithelial electrical resistance (TEER) measurements and permeability to several control compounds. TEER values are measured in PAB using an epithelial voltammeter with an Endohm-12 electrode (World Precision Instruments). The control compounds used to assess the suitability of a batch of cells are digoxin, lucifer yellow, atenolol, and propranolol. For each monolayer tested the TEER value and the permeability coefficient for the different control compounds have to be within a specified range before a given batch of cells may be certified as "acceptable" .

Pre-Experiment Batch Acceptance Criteria:

Atenolol Papp (x $10^{-6}$ cm/s) < 0.5

Propranolol Papp (x $10^{-6}$ cm/s) 15 - 25

Lucifer Yellow Papp (x $10^{-6}$ cm/s) < 0.4

Digoxin Ratio Papp (B-A) / Papp (A-B) > 3

Following the acceptance of a batch, the TEER value of each monolayer intended to be used in permeability studies is measured prior to inclusion in the study. Monolayers with TEER values in the 450-650 $\Omega.cm^2$ range are included in the permeability study, and those with TEER values outside this range are discarded.

Test Method:

Uni-directional permeability of pro-drug (compound according to the invention) and conversion assessment of pro-drug to drug (the ester of alkyl fumarate corresponding to the compound according to the invention) is performed as follows:

Caco-2 cell monolayers are grown to confluence on collagen-coated Transwell® polycarbonate filters as outlined above, and used 21 to 28 days post-seeding. The permeability assay buffer is Hank's Balanced Salt Solution containing 10 mM HEPES and 15 mM glucose at a pH of 7.4. The dosing solution concentration is e.g. 100 μM of the pro-drug in assay buffer. The receiver (basolateral) side contains 1% bovine serum albumin (BSA) in modified Hank's buffer. Cells are dosed on the apical (donor) side and incubated at 37°C with 5% $CO_2$ in a humidified incubator. Each determination is performed in duplicate (in duplicate wells). At t=90 minutes, the receiver as well as the donor sides are sampled, and both samples are analyzed with respect to the amount of pro-drug and the amount of converted drug (assessing the apical-to-basolateral transport of pro-drug (A to B)). Lucifer yellow flux is also measured for each monolayer after being subjected to the test articles to ensure that no damage is inflicted to the cell monolayers during the flux period. Both receiver and donor samples are assayed by liquid chromatography (LC)/mass spectrometer (MS) with an appropriate standard curve.

Analytical:

All samples are analyzed by LC/MS using a PE SCIEX API 150 or API 2000 mass spectrometer. The chromatographic system consists of two Perkin Elmer Series 200 micro LC pumps and a Perkin Elmer Series 200 autosampler.

Permeability Calculation:

The apparent permeability (Papp) and percent recovery can be calculated according to the following equation: Papp = ($dC_r$ /$dt$) x $V_r$/(A x $C_0$) where, $dC_r$ /dt is the cumulative concentration in the receiver compartment versus time in μM $s^{-1}$. $V_r$ is the volume of the receiver compartment (e.g. 1.5 $cm^3$). A is the area of the cell monolayer (1.13 $cm^2$ for 12-well Transwell). $C_0$ is the concentration of the dosing solution in μM. A linear fit of the cumulative concentration versus time is made to determine $dC_r$ /dt. The origin is not included in the fit.

Further embodiments according to the invention:

Embodiment 1. A compound of the general formula (I) or (II)

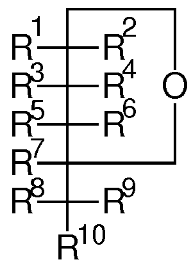

(I)

or

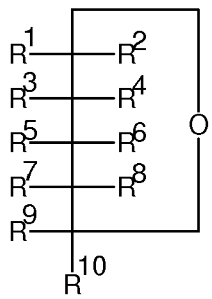

(II)

wherein $R^1$, $R^2$, $R^3$, $R^4$, $R^5$, $R^6$, $R^7$, $R^8$ and $R^9$ are the same or different and each is selected from the group consisting of hydrogen, hydroxyl, methoxy, methoxymethoxy, benzyloxy, acetate, acetonide and X, and
$R^{10}$ is selected from the group consisting of hydrogen, hydroxyl, $-CH_2OH$, $-CH_3$, X and $-CH_2$-X,
wherein
X is -OOC-CH=CH-COOR (trans) wherein R is $C_{1-5}$alkyl;
with the proviso that at least one of $R^1$, $R^2$, $R^3$, $R^4$, $R^5$, $R^6$, $R^7$, $R^8$, $R^9$ and $R^{10}$ is X or $R^{10}$ is $-CH_2X$,

and any enantiomers, racemates, diastereomers, anomers or tautomeric forms or mixtures thereof.

Embodiment 2. The compound according to embodiment 1, wherein $R^1$, $R^2$, $R^3$, $R^4$, $R^5$, $R^6$, $R^7$, $R^8$ and $R^9$ are the same or different and each is selected from the group consisting of hydrogen, hydroxyl and X.

Embodiment 3. The compound according to any one of the embodiments 1-2, which is a compound of general formula I.

Embodiment 4. The compound according to any one of the embodiments 1-2, which is a compound of general formula II.

Embodiment 5. The compound according to any one of the embodiments 1-4, which is a D-alpha anomer or a D-beta anomer or a mixture thereof.

Embodiment 6. The compound according to any one of the embodiments 1-5, which is a D-alpha anomer.

Embodiment 7. The compound according to embodiment 1, wherein five or six selected from the group consisting of $R^1$, $R^2$, $R^3$, $R^4$, $R^5$, $R^6$, $R^7$, $R^8$, $R^9$ and $R^{10}$ are hydrogen.

Embodiment 8. The compound according to any one of the preceding embodiments, wherein R is methyl.

Embodiment 9. The compound according to any one of the preceding embodiments, wherein $R^2$ is hydroxyl.

Embodiment 10. The compound according to any one of the preceding embodiments, wherein $R^{10}$ is $-CH_2$-X.

Embodiment 11. The compound according to any one of the preceding embodiments, wherein $R^1$ is hydrogen.

Embodiment 12. The compound according to any one of the preceding embodiments, wherein either $R^3$ or $R^4$ is hydrogen and the other is hydroxyl.

Embodiment 13. The compound according to any one of the preceding embodiments, wherein either $R^5$ or $R^6$ is hydrogen and the other is hydroxyl.

Embodiment 14. The compound according to any one of the preceding embodiments, wherein either $R^7$ or $R^8$ is hydrogen and the other is hydroxyl.

Embodiment 15. The compound according to embodiment 1 which is selected from the group consisting of arabinofuranosyl-$C_{1-5}$alkyl-fumarate, galactofuranosyl-$C_{1-5}$alkyl-fumarate, glucofuranosyl-$C_{1-5}$alkyl-fumarate, mannofuranosyl-$C_{1-5}$alkyl-fumarate, xylofuranosyl-$C_{1-5}$alkyl-fumarate, fructofuranosyl-$C_{1-5}$alkyl-fumarate, arabinopyranosyl-$C_{1-5}$alkyl-fumarate, galactopyranosyl-$C_{1-5}$alkyl-fumarate, glucopyranosyl-$C_{1-5}$alkyl-fumarate, mannopyranosyl-$C_{1-5}$alkyl-fumarate, xylopyranosyl-$C_{1-5}$alkyl-fumarate and fructopyranosyl-$C_{1-5}$alkyl-fumarate.

Embodiment 16. The compound according to embodiment 15, wherein $C_{1-5}$alkyl is methyl.

Embodiment 17. The compound according to embodiment 1, the compound is selected from the group consisting of

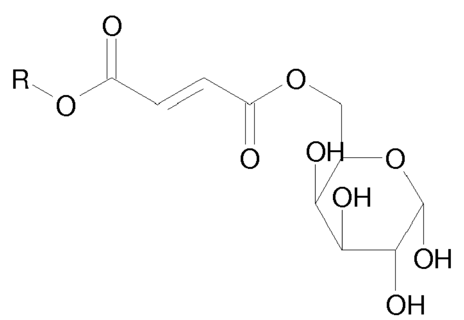

α-D-galactopyranose, 6-( $C_{1-5}$alkyl fumarate),

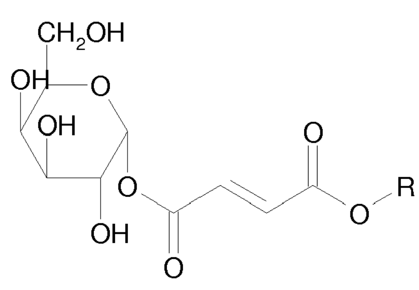

α-D-galactopyranose, 1-( $C_{1-5}$alkyl fumarate),

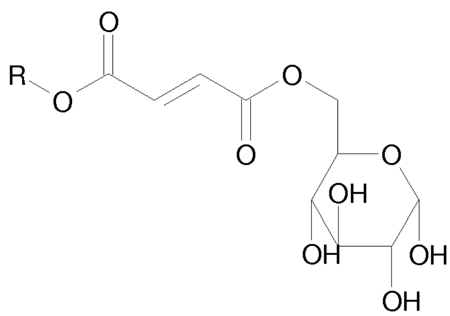

α-D-glucopyranose, 6-( $C_{1-5}$alkyl fumarate),

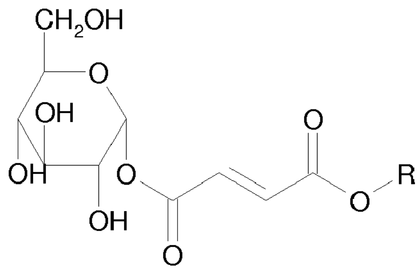

α-D-glucopyranose, 1-( $C_{1-5}$alkyl fumarate),

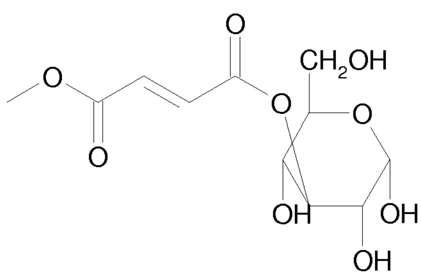

α-D-glucopyranose, 3-( $C_{1-5}$alkyl fumarate),

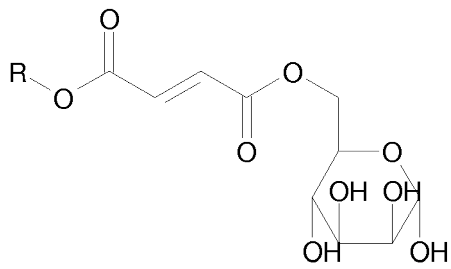

α-D-mannopyranose, 6-( $C_{1-5}$alkyl fumarate),

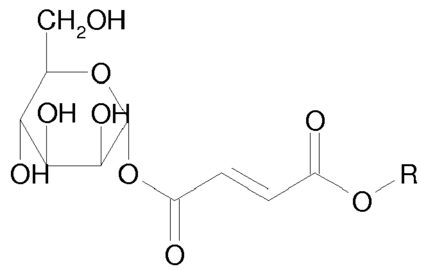

α-D-mannopyranose, 1-( $C_{1-5}$alkyl fumarate),

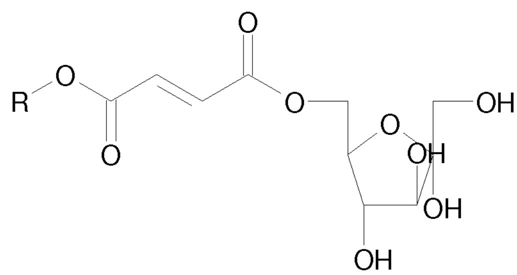

α-D-fructofuranose,6-( $C_{1-5}$alkyl fumarate),

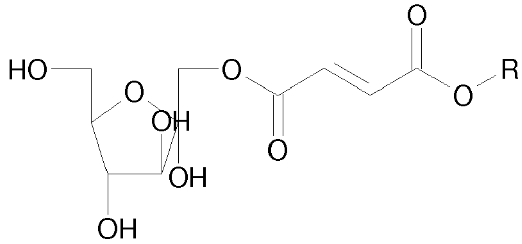

α-D-fructofuranose,1-( $C_{1-5}$alkyl fumarate),

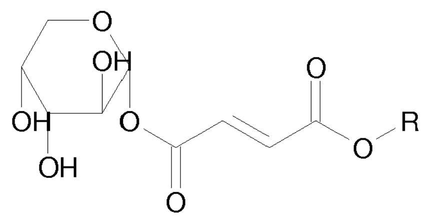

α-D-arabinopyranose,1-( $C_{1-5}$alkyl fumarate),

and

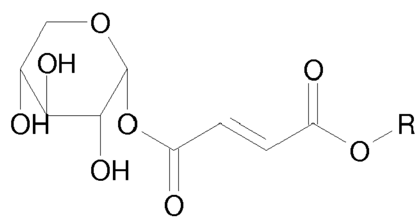

α-D-xylopyranose,1-( $C_{1-5}$alkyl fumarate),

wherein R is $C_{1-5}$alkyl.

Embodiment 18. The compound according to embodiment 17, wherein R is methyl.

Embodiment 19. A composition comprising a compound according to any one of the embodiments 1-18 in combination with di($C_{1-5}$)alkylester of fumaric acid.

Embodiment 20. A composition comprising a compound according to any one of the embodiments 1-18 in combination with a mono($C_{1-5}$)alkylester of fumaric acid, optionally in the form of a pharmaceutically acceptable salt.

Embodiment 21. A compound according to any one of the embodiments 1-18 or a composition according to any one of the embodiments 19-20 for use in medicine.

Embodiment 22. A compound according to any one of the embodiments 1-18 or a composition according to any one of the embodiments 19-20 for combating tissue degenerative processes.

Embodiment 23. A compound according to any one of the embodiments 1-18 or a composition according to any one of the embodiments 19-20 for use in the treatment of one or more of the following conditions psoriasis; psoriatic arthritis; neurodermatitis; atopic dermatitis; inflammatory bowel disease; and/or autoimmune diseases.

Embodiment 24. A compound according to any one of the embodiments 1-18 or a composition according to any one of the embodiments 19-20 for use in the treatment of one or more of the following conditions pain; sarcoidosis; necrobiosis lipoidica; and/or granuloma annulare: or for use in prevention of rejection following organ transplantation.

Embodiment 25. A compound according to any one of the embodiments 1-18 or a composition according to any

one of the embodiments 19-20 for use in the treatment of one or more of the following conditions lupus nephritis, myasthenia gravis, uveitis, refractory uveitis, vernal conjunctivitis, pemphigus vulgaris, and/or scleroderma.

Embodiment 26. A compound according to any one of the embodiments 1-18 or a composition according to any one of the embodiments 19-20 for use in the treatment of multiple sclerosis.

Embodiment 27. A compound according to any one of the embodiments 1-18 or a composition according to any one of the embodiments 19-20 for use in the treatment of psoriasis.

Embodiment 28. A compound according to any one of the embodiments 1-18 or a composition according to any one of the embodiments 19-20 for use in the treatment of psoriatic arthritis.

Embodiment 29. A pharmaceutical composition comprising a compound as defined in any one of the embodiments 1-18.

Embodiment 30. The pharmaceutical composition according to embodiment 29 in the form of a controlled release composition.

Embodiment 31. A method of treating one or more conditions selected from the group consisting of psoriasis, psoriatic arthritis, neurodermatitis, atopic dermatitis, inflammatory bowel disease, autoimmune diseases, pain, organ transplantation (prevention of rejection), sarcoidosis, necrobiosis lipoidica, granuloma annulare, lupus nephritis, myasthenia gravis, uveitis, refractory uveitis, vernal conjunctivitis, pemphigus vulgaris, and scleroderma, which method comprises administering orally to a patient in need thereof, an effective dosage of a compound according to any one of the embodiments 1-18.

Embodiment 32. The method according to embodiment 31, wherein the autoimmune disease is multiple sclerosis.

Embodiment 33. The method according to embodiment 31, wherein the condition is psoriasis.

Embodiment 34. The method according to embodiment 31, wherein the condition is psoriatic arthritis.

Embodiment 35. Use of a compound according to any one of the embodiments 1-18 or a composition according to any one of the embodiments 19-20 for the manufacture of a pharmaceutical composition for use in the treatment of one or more conditions selected from the group consisting of psoriasis, psoriatic arthritis, neurodermatitis, atopic dermatitis, inflammatory bowel disease, autoimmune diseases , pain, organ transplantation (prevention of rejection), sarcoidosis, necrobiosis lipoidica, granuloma annulare, lupus nephritis, myasthenia gravis, uveitis, refractory uveitis, vernal conjunctivitis, pemphigus vulgaris, and/or scleroderma.

Embodiment 36. The use according to embodiment 35, wherein the autoimmune disease is multiple sclerosis.

Embodiment 37. The use according to embodiment 35, wherein the condition is psoriasis.

Embodiment 38. The use according to embodiment 35, wherein the condition is psoriatic arthritis.

Further embodiments according to the invention:

Embodiment 1a. A compound of the general formula (I) or (II)

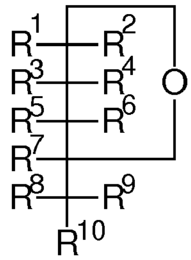

(I)

or

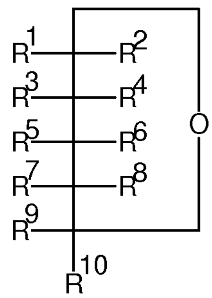

(II)

wherein $R^1$, $R^2$, $R^3$, $R^4$, $R^5$, $R^6$, $R^7$, $R^8$ and $R^9$ are the same or different and each is selected from the group consisting of hydrogen, hydroxyl, methoxy, methoxymethoxy, benzyloxy, acetate, acetonide and X, and $R^{10}$ is selected from the group consisting of hydrogen, hydroxyl, $-CH_2OH$, $-CH_3$, X and $-CH_2$-X, wherein

X is -OOC-CH=CH-COOR (trans) wherein R is $C_{1-5}$alkyl;

with the proviso that at least one of $R^1$, $R^2$, $R^3$, $R^4$, $R^5$, $R^6$, $R^7$, $R^8$, $R^9$ and $R^{10}$ is X or $R^{10}$ is $-CH_2X$,

and any enantiomers, racemates, diastereomers, anomers or tautomeric forms or mixtures thereof, with the proviso that the methyl ester of methyl-4-O-fumaryl-alfa-L-diginoside is disclaimed, and with the further proviso that compounds of the general formula (I) or (II)

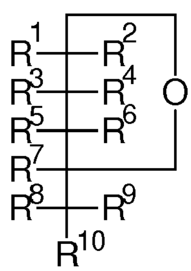

(I)

or

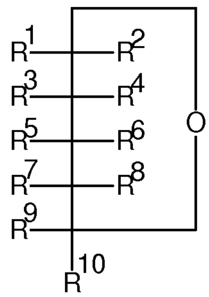

(II)

wherein $R^1$, $R^2$, $R^3$, $R^4$, $R^5$, $R^6$, $R^7$, $R^8$ and $R^9$ are the same or different and each is selected from the group consisting of hydrogen, hydroxyl, and X, and

$R^{10}$ is selected from the group consisting of hydrogen, hydroxyl, $-CH_2OH$, $-CH_3$, X and $-CH_2$-X,

wherein

X is -OOC-CH=CH-COOR (trans) wherein R is $C_{1-5}$alkyl ;

with the proviso that at least one of $R^1$, $R^2$, $R^3$, $R^4$, $R^5$, $R^6$, $R^7$, $R^8$, $R^9$ and $R^{10}$ is X or $R^{10}$ is- $CH_2X$,

and any enantiomers, racemates, diastereomers, anomers or tautomeric forms or mixtures thereof, are disclaimed.

Embodiment 2a. The compound according to embodiment 1a, which is a compound of general formula I.

Embodiment 3a. The compound according to embodiment 1a, which is a compound of general formula II.

Embodiment 4a. The compound according to any one of the embodiments 1a-3a, which is a D-alpha anomer or a D-beta anomer or a mixture thereof.

Embodiment 5a. The compound according to any one of the embodiments 1a-4a, which is a D-alpha anomer.

Embodiment 6a. The compound according to embodiment 1a, wherein five or six selected from the group consisting of $R^1$, $R^2$, $R^3$, $R^4$, $R^5$, $R^6$, $R^7$, $R^8$, $R^9$ and $R^{10}$ are hydrogen.

Embodiment 7a. The compound according to any one of the preceding embodiments, wherein R is methyl.

Embodiment 8a. The compound according to any one of the preceding embodiments, wherein $R^2$ is hydroxyl.

Embodiment 9a. The compound according to any one of the preceding embodiments, wherein $R^{10}$ is $-CH_2$-X.

Embodiment 10a. The compound according to any one of the preceding embodiments, wherein $R^1$ is hydrogen.

Embodiment 11a. The compound according to any one of the preceding embodiments, wherein either $R^3$ or $R^4$ is hydrogen and the other is hydroxyl.

Embodiment 12a. The compound according to any one of the preceding embodiments, wherein either $R^5$ or $R^6$ is hydrogen and the other is hydroxyl.

Embodiment 13a. The compound according to any one of the preceding embodiments, wherein either $R^7$ or $R^8$ is hydrogen and the other is hydroxyl.

Embodiment 14a. A composition comprising a compound according to any one of the embodiments 1a-13a or the methyl ester of methyl-4-O-fumaryl-alfa-L-diginoside in combination with di($C_{1-5}$)alkylester of fumaric acid.

Embodiment 15a. A composition comprising a compound according to any one of the embodiments 1a-13a or the methyl ester of methyl-4-O-fumaryl-alfa-L-diginoside in combination with a mono($C_{1-5}$)alkylester of fumaric acid, optionally in the form of a pharmaceutically acceptable salt.

Embodiment 16a. A compound according to any one of the embodiments 1a-13a or the methyl ester of methyl-4-O-fumaryl-alfa-L-diginoside or a composition according to any one of the embodiments 14a-15a for use in medicine.

Embodiment 17a. A compound according to any one of the embodiments 1a-13a or the methyl ester of methyl-4-O-fumaryl-alfa-L-diginoside or a composition according to any one of the embodiments 14a-15a for combating tissue degenerative processes.

Embodiment 18a. A compound according to any one of the embodiments 1a-13a or the methyl ester of methyl-4-O-fumaryl-alfa-L-diginoside or a composition according to any one of the embodiments 14a-15a for use in the treatment of one or more of the following conditions psoriasis; psoriatic arthritis; neurodermatitis; atopic dermatitis; inflammatory bowel disease; and/or autoimmune diseases.

Embodiment 19a. A compound according to any one of the embodiments 1a-13a or the methyl ester of methyl-4-O-fumaryl-alfa-L-diginoside or a composition according to any one of the embodiments 14a-15a for use in the treatment of one or more of the following conditions pain; sarcoidosis; necrobiosis lipoidica; and/or granuloma annulare: or for use in prevention of rejection following organ transplantation.

Embodiment 20a. A compound according to any one of the embodiments 1a-13a or the methyl ester of methyl-4-O-fumaryl-alfa-L-diginoside or a composition according to any one of the embodiments 14a-15a for use in the treatment of one or more of the following conditions lupus nephritis, myasthenia gravis, uveitis, refractory uveitis, vernal conjunctivitis, pemphigus vulgaris, and/or scleroderma.

Embodiment 21a. A compound according to any one of the embodiments 1a-13a or the methyl ester of methyl-4-O-fumaryl-alfa-L-diginoside or a composition according to any one of the embodiments 14a-15a for use in the treatment of multiple sclerosis.

Embodiment 22a. A compound according to any one of the embodiments 1a-13a or the methyl ester of methyl-4-O-fumaryl-alfa-L-diginoside or a composition according to any one of the embodiments 14a-15a for use in the treatment of psoriasis.

Embodiment 23a. A compound according to any one of the embodiments 1a-13a or the methyl ester of methyl-4-O-fumaryl-alfa-L-diginoside or a composition according to any one of the embodiments 14a-15a for use in the treatment of psoriatic arthritis.

Embodiment 24a. A pharmaceutical composition comprising a compound as defined in any one of the embodiments 1a-13a or the methyl ester of methyl-4-O-fumaryl-alfa-L-diginoside.

Embodiment 25a. The pharmaceutical composition according to embodiment 24a in the form of a controlled release composition.

Embodiment 26a. Use of a compound according to any one of the embodiments 1a-13a or the methyl ester of methyl-4-O-fumaryl-alfa-L-diginoside or a composition according to any one of the embodiments 14a-15a for the manufacture of a pharmaceutical composition for use in the treatment of one or more conditions selected from the group consisting of psoriasis, psoriatic arthritis, neurodermatitis, atopic dermatitis, inflammatory bowel disease, autoimmune diseases , pain, organ transplantation (prevention of rejection), sarcoidosis, necrobiosis lipoidica, granuloma annulare, lupus nephritis, myasthenia gravis, uveitis, refractory uveitis, vernal conjunctivitis, pemphigus vulgaris, and/or scleroderma.

Embodiment 27a. The use according to embodiment 26a, wherein the autoimmune disease is multiple sclerosis.

Embodiment 28a. The use according to embodiment 26a, wherein the condition is psoriasis.

Embodiment 29a. The use according to embodiment 26a, wherein the condition is psoriatic arthritis.

## Claims

1. A compound of the general formula (I) or (II)

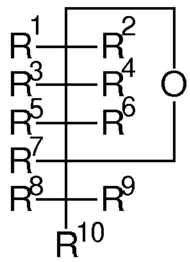

(I)

or

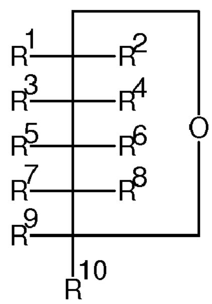

(II)

wherein $R^1$, $R^2$, $R^3$, $R^4$, $R^5$, $R^6$, $R^7$, $R^8$ and $R^9$ are the same or different and each is selected from the group consisting of hydrogen, hydroxyl, methoxy, methoxymethoxy, benzyloxy, acetate, acetonide and X, and $R^{10}$ is selected from the group consisting of hydrogen, hydroxyl, $-CH_2OH$, $-CH_3$, X and $-CH_2-X$,
wherein
X is -OOC-CH=CH-COOR (trans) wherein R is $C_{1-5}$alkyl;
with the proviso that at least one of $R^1$, $R^2$, $R^3$, $R^4$, $R^5$, $R^6$, $R^7$, $R^8$, $R^9$ and $R^{10}$ is X or $R^{10}$ is $-CH_2X$,

and any enantiomers, racemates, diastereomers, anomers or tautomeric forms or mixtures thereof,
with the proviso that the methyl ester of methyl-4-O-fumaryl-alfa-L-diginoside is disclaimed, and
with the further proviso that compounds of the general formula (I) or (II)

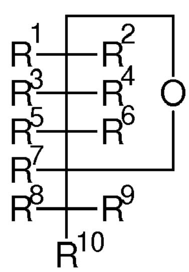

(I)

or

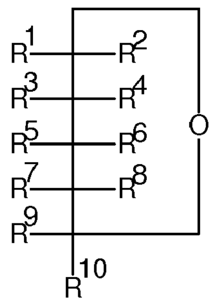

(II)

wherein $R^1$, $R^2$, $R^3$, $R^4$, $R^5$, $R^6$, $R^7$, $R^8$ and $R^9$ are the same or different and each is selected from the group consisting of hydrogen, hydroxyl, and X, and
$R^{10}$ is selected from the group consisting of hydrogen, hydroxyl, $-CH_2OH$, $-CH_3$, X and $-CH_2$-X,
wherein
X is -OOC-CH=CH-COOR (trans) wherein R is $C_{1-5}$alkyl;
with the proviso that at least one of $R^1$, $R^2$, $R^3$, $R^4$, $R^5$, $R^6$, $R^7$, $R^8$, $R^9$ and $R^{10}$ is X or $R^{10}$ is- $CH_2X$,

and any enantiomers, racemates, diastereomers, anomers or tautomeric forms or mixtures thereof, are disclaimed.

**2.** The compound according to claim 1, which is a compound of general formula I.

**3.** The compound according to claim 1, which is a compound of general formula II.

**4.** The compound according to any one of the claims 1-3, which is a D-alpha anomer or a D-beta anomer or a mixture thereof.

**5.** The compound according to any one of the claims 1-4, which is a D-alpha anomer.

**6.** The compound according to claim 1, wherein five or six selected from the group consisting of $R^1$, $R^2$, $R^3$, $R^4$, $R^5$, $R^6$, $R^7$, $R^8$, $R^9$ and $R^{10}$ are hydrogen.

**7.** The compound according to any one of the preceding claims, wherein R is methyl.

**8.** The compound according to any one of the preceding claims, wherein $R^2$ is hydroxyl.

**9.** The compound according to any one of the preceding claims, wherein $R^{10}$ is $-CH_2$-X.

**10.** The compound according to any one of the preceding claims, wherein $R^1$ is hydrogen.

**11.** A composition comprising a compound according to any one of the claims 1-10 or the methyl ester of methyl-4-O-fumaryl-alfa-L-diginoside in combination with di($C_{1-5}$)alkylester of fumaric acid.

**12.** A composition comprising a compound according to any one of the claims 1-10 or the methyl ester of methyl-4-O-fumaryl-alfa-L-diginoside in combination with a mono($C_{1-5}$)alkylester of fumaric acid, optionally in the form of a pharmaceutically acceptable salt.

**13.** A compound according to any one of the claims 1-10 or the methyl ester of methyl-4-O-fumaryl-alfa-L-diginoside or a composition according to any one of the claims 11-12 for use in medicine.

**14.** A pharmaceutical composition comprising a compound as defined in any one of the claims 1-10 or the methyl ester of methyl-4-O-fumaryl-alfa-L-diginoside.

**15.** Use of a compound according to any one of the claims 1-10 or the methyl ester of methyl-4-O-fumaryl-alfa-L-diginoside or a composition according to any one of the claims 11-12 for the manufacture of a pharmaceutical composition for use in the treatment of one or more conditions selected from the group consisting of psoriasis,

psoriatic arthritis, neurodermatitis, atopic dermatitis, inflammatory bowel disease, autoimmune diseases , pain, organ transplantation (prevention of rejection), sarcoidosis, necrobiosis lipoidica, granuloma annulare, lupus nephritis, myasthenia gravis, uveitis, refractory uveitis, vernal conjunctivitis, pemphigus vulgaris, and/or scleroderma.

Europäisches Patentamt
European Patent Office
Office européen des brevets

# EUROPEAN SEARCH REPORT

Application Number
EP 10 15 1082

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | TETRAHEDRON LETTERS , 25(18), 1941-4 CODEN: TELEAY; ISSN: 0040-4039, 1984, XP002101927<br>* the whole document *<br>-& DATABASE CA [Online] CHEMICAL ABSTRACTS SERVICE, COLUMBUS, OHIO, US; 16 November 1984 (1984-11-16), KAWAI, HIROYUKI ET AL: "Studies on arugomycin, a new anthracycline antibiotic. Part II. Structural elucidation of arugomycin" XP002396501 retrieved from STN Database accession no. 101:152215<br>* abstract *<br>----- | 1-15 | INV.<br>C07H13/04<br>C07D309/06<br>A61K31/351<br>A61K31/341<br>A61K31/7028<br>A61K31/7024 |
| A | DE 25 30 372 A1 (SCHWECKENDIEK,WALTER,DR) 13 January 1977 (1977-01-13)<br>----- | 1-15 | |
| A | US 6 509 376 B1 (JOSHI RAJENDRA KUMAR ET AL) 21 January 2003 (2003-01-21)<br>----- | 1-15 | TECHNICAL FIELDS SEARCHED (IPC) |
| A | US 6 277 882 B1 (JOSHI RAJENDRA K ET AL) 21 August 2001 (2001-08-21)<br>----- | 1-15 | C07H<br>C07D<br>A61K |

The present search report has been drawn up for all claims

1

| Place of search | Date of completion of the search | Examiner |
|---|---|---|
| Munich | 30 March 2010 | Klein, Didier |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

**ANNEX TO THE EUROPEAN SEARCH REPORT ON EUROPEAN PATENT APPLICATION NO.** EP 10 15 1082

This annex lists the patent family members relating to the patent documents cited in the above-mentioned European search report.
The members are as contained in the European Patent Office EDP file on
The European Patent Office is in no way liable for these particulars which are merely given for the purpose of information.

30-03-2010

| Patent document cited in search report | | Publication date | Patent family member(s) | | | Publication date |
|---|---|---|---|---|---|---|
| DE 2530372 | A1 | 13-01-1977 | NONE | | | |
| US 6509376 | B1 | 21-01-2003 | AT | 242633 | T | 15-06-2003 |
| | | | AU | 752733 | B2 | 26-09-2002 |
| | | | AU | 1157200 | A | 13-06-2000 |
| | | | BG | 64837 | B1 | 30-06-2006 |
| | | | BG | 105396 | A | 31-12-2001 |
| | | | BR | 9914606 | A | 03-07-2001 |
| | | | CA | 2346438 | A1 | 02-06-2000 |
| | | | CN | 1325302 | A | 05-12-2001 |
| | | | CN | 1615839 | A | 18-05-2005 |
| | | | CZ | 20010939 | A3 | 15-05-2002 |
| | | | DE | 19853487 | A1 | 25-05-2000 |
| | | | DK | 1131065 | T3 | 25-08-2003 |
| | | | EE | 200100266 | A | 16-12-2002 |
| | | | WO | 0030622 | A2 | 02-06-2000 |
| | | | EP | 1131065 | A2 | 12-09-2001 |
| | | | ES | 2195664 | T3 | 01-12-2003 |
| | | | HK | 1042242 | A1 | 19-08-2005 |
| | | | HU | 0103182 | A2 | 28-01-2002 |
| | | | JP | 2002530324 | T | 17-09-2002 |
| | | | JP | 2005097323 | A | 14-04-2005 |
| | | | JP | 2009256384 | A | 05-11-2009 |
| | | | MX | PA01005007 | A | 24-04-2002 |
| | | | NO | 20011242 | A | 18-05-2001 |
| | | | NZ | 510247 | A | 31-10-2003 |
| | | | PL | 348147 | A1 | 06-05-2002 |
| | | | PT | 1131065 | E | 31-10-2003 |
| | | | RU | 2215524 | C2 | 10-11-2003 |
| | | | SK | 5762001 | A3 | 05-03-2002 |
| | | | TR | 200101204 | T2 | 21-09-2001 |
| | | | US | 2003018072 | A1 | 23-01-2003 |
| | | | ZA | 200202335 | A | 27-08-2003 |
| US 6277882 | B1 | 21-08-2001 | AT | 219936 | T | 15-07-2002 |
| | | | AU | 745281 | B2 | 21-03-2002 |
| | | | AU | 2159399 | A | 18-10-1999 |
| | | | BG | 64744 | B1 | 28-02-2006 |
| | | | BG | 104566 | A | 31-01-2001 |
| | | | BR | 9815772 | A | 21-11-2000 |
| | | | CA | 2283915 | A1 | 30-09-1999 |
| | | | CN | 1284870 | A | 21-02-2001 |
| | | | CZ | 20003086 | A3 | 15-11-2000 |
| | | | DE | 19814358 | A1 | 07-10-1999 |
| | | | DK | 1059920 | T3 | 14-10-2002 |
| | | | EE | 200000440 | A | 17-12-2001 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 10 15 1082

This annex lists the patent family members relating to the patent documents cited in the above-mentioned European search report.
The members are as contained in the European Patent Office EDP file on
The European Patent Office is in no way liable for these particulars which are merely given for the purpose of information.

30-03-2010

| Patent document cited in search report | Publication date | Patent family member(s) | Publication date |
|---|---|---|---|
| US 6277882 B1 | | WO 9949858 A1 | 07-10-1999 |
| | | EP 1059920 A1 | 20-12-2000 |
| | | ES 2179551 T3 | 16-01-2003 |
| | | HK 1035134 A1 | 11-03-2005 |
| | | HU 0103234 A2 | 28-01-2002 |
| | | IL 136835 A | 17-05-2005 |
| | | JP 3527890 B2 | 17-05-2004 |
| | | JP 2002509883 T | 02-04-2002 |
| | | NO 20002872 A | 06-06-2000 |
| | | NZ 505341 A | 26-04-2002 |
| | | PL 341240 A1 | 26-03-2001 |
| | | PT 1059920 E | 29-11-2002 |
| | | RU 2197233 C2 | 27-01-2003 |
| | | SK 7922000 A3 | 18-01-2001 |
| | | TR 200001829 T2 | 21-11-2000 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6277882 B
- US 6355676 B
- US 6509376 B
- US 5424332 A
- US 6359003 B
- US 4959389 A
- US 20040038889 A
- WO 8901830 A
- DK 2005000648 W
- WO 03004001 A
- WO 03080034 A
- US 6537584 B, Zentner
- US 5484610 A, Bae


### Non-patent literature cited in the description


- **Litjens et al.** *Br. J. Clin. Pharmacol.,* 2004, vol. 58 (4), 429-432
- Treatment of disseminated granuloma annulare with fumaric acid esters. *BMC Dermatology,* 2002, vol. 2 (5
- **Altmeyer et al.** *J. Am. Acad. Dermatol.,* 1994
- **Altmeyer PJ et al.** Antipsoriatic effect of fumaric acid derivatives. Results of a multicenter double-blind study in 100 patients. *J. Am. Acad. Dermatol.,* 1994, vol. 30, 977-81
- Flushing and flushing syndromes, rosacea and perioral dermatitis. **Greaves MW et al.** Rook/Wilkinson/Ebling textbook of dermatology. Blackwell Scientific, 1998, vol. 3, 2099-2104
- **O'toole et al.** *Cancer,* 2000, vol. 88 (4), 770-776
- **Werdenberg et al.** *BioPharm. Drug Dispos.,* 2003, vol. 24, 259-273
- **K.S. Rao ; S.H. Mishra.** *J. Ethnopharmacology,* 1998, vol. 60 (3), 207-213
- **Spatz ; Stone.** *J. Org. Chem.,* 1958, vol. 23 (10), 1559-1560
- **A. Jakas ; V. Magnus ; S. Horvat ; G. Sandberg.** *J. Labell. Comp. Pharm.,* 1993, vol. 33 (10), 933-939
- **K. Lohith ; S. Divakar.** *J. Biotechnol.,* 2005, vol. 117, 49-56
- **Najarian DJ ; Gottlieb AB.** Connections between psoriasis and Crohn's disease. *J Am Acad Dermatol.,* June 2003, vol. 48 (6), 805-21
- **Ojetti V et al.** High prevalence of celiac disease in psoriasis. *Am J Gastroenterol.,* November 2003, vol. 98 (11), 2574-5
- **Gupta MA ; Gupta AK.** Psychiatric and psychological co-morbidity in patients with dermatologic disorders: epidemiology and management. *Am J Clin Dermatol.,* 2003, vol. 4 (12), 833-42
- **Mallbris L et al.** Psoriasis phenotype at disease onset: clinical characterization of 400 adult cases. *J Invest Dermatol.,* March 2005, vol. 124 (3), 499-504